# EUROPEAN PATENT SPECIFICATION

(11) **EP 2 724 729 B1**
(45) Date of publication and mention of the grant of the patent: **26.12.2018**
(21) Application number: 11868386.1
(22) Date of filing: 21.06.2011
(51) Int. Cl.: A61K 45/06, A61K 31/7088, A61K 31/713, A61K 38/45, A61K 45/00, A61K 48/00, A61P 35/00, A61P 43/00, A61K 31/52, A61K 31/7105, A61K 31/711, C12N 15/113

(54) **APOPTOSIS-INDUCING AGENT**
APOPTOSEAUSLÖSER
AGENT INDUCTEUR D'APOPTOSE

(43) Date of publication of application: 30.04.2014
(73) Proprietor: Nitto Denko Corporation, Ibaraki-shi, Osaka 567-8680 (JP)
(72) Inventor: NIITSU, Yoshiro, Sapporo-shi Hokkaido 064-0823 (JP); NISHITA, Hiroki, Sapporo-shi Hokkaido 060-0005 (JP)
(74) Representative: Hoffmann Eitle
(86) International application number: PCT/JP2011/064163
(87) International publication number: WO 2012/176282

(56) References cited:
- WO-A1-96/40205
- WO-A2-2006/078774
- US-A1- 2013 064 815
- Nishita et al.: "Abstract 1065: Regulation of autophagy and MAPK signaling by glutathione S-transferase-pi in KRAS mutated cancer cells", Cancer Research, 15 April 2011 (2011-04-15), XP002736462, DOI: 10.1158/1538-7445.AM2011-1065 Retrieved from the Internet: URL:http://cancerres.aacrjournals.org/cont ent/71/8_Supplement/1065.abstract [retrieved on 2015-02-24]
- BAN NORIYOSHI ET AL: "Transfection of glutathione S-transferase (gST)-pi antisense complementary DNA increases the sensitivity of a colon cancer cell line to adriamycin, cisplatin, melphalan, and etoposide", CANCER RESEARCH, vol. 56, no. 15, 1996, pages 3577-3582, XP002736463, ISSN: 0008-5472
- KOJI MIYANISHI ET AL: "Glutathione S-transferase-[pi] overexpression is closely associated with K-ras mutation during human colon carcinogenesis", GASTROENTEROLOGY, vol. 121, no. 4, 1 October 2001 (2001-10-01), pages 865-874, XP055087152, ISSN: 0016-5085, DOI: 10.1053/gast.2001.27982
- AMARAVADI RAVI K ET AL: "Autophagy inhibition enhances therapy-induced apoptosis in a Myc-induced model of lymphoma", JOURNAL OF CLINICAL INVESTIGATION, vol. 117, no. 2, February 2007 (2007-02), pages 326-336, XP55171973, ISSN: 0021-9738
- TETSUJI TAKAYAMA ET AL.: 'GST-n o Hyoteki toshita Daichogan no Kagaku Yobo' FRONTIERS IN GASTROENTEROLOGY vol. 15, no. L, 2010, pages 11 - 17, XP008172562
- 'Autophagy Inhibition Enhances Apoptosis Induced by Ginsenoside Rkl in Hepatocellular Carcinoma Cells' BIOSCIENCE, BIOTECHNOLOGY, AND BIOCHEMISTRY vol. 73, no. 10, 2009, pages 2183 - 2189, XP009155223
- HOKAIWADO N. ET AL.: 'Glutathione S-transferase Pi mediates proliferation of androgen-independent prostate cancer cells' CARCINOGENESIS vol. 29, no. 6, 2008, pages 1134 - 1138, XP055141304
- SATOHIRO TAKANASHI ET AL.: 'Daichogan Hatsugan Katei ni Okeru GSTpi no MAP kinase Chosetsu Inshi to shite no Igi' PROCEEDINGS OF THE JAPANESE CANCER ASSOCIATION vol. 66, 2007, page 181, XP008172563
- KEISUKE MIYAZAWA ET AL.: 'Vitamin K2 ni yoru Hakketsubyo Saibo no Autophagy/Apoptosis Yudo' RINSHO KETSUEKI vol. 48, no. 9, 2007, page 1097, XP008172247
- SACHIKO TSUKAMOTO ET AL.: 'Tenkai suru Proteasome Sogaizai Kenkyu' EXPERIMENTAL MEDICINE vol. 26, no. 2, 2008, pages 242 - 247, XP008172561

## Description

### [Technical Field]

The present description relates to novel use of GST-π and a suppressing agent thereof, a novel apoptosis-inducing agent, a pharmaceutical composition containing the apoptosis-inducing agent, and a novel therapeutic method for a disease associated with abnormal apoptosis.

### [Background Art]

Cancer is one of the most important and troublesome diseases that confront mankind, and an enormous amount of research effort into the treatment thereof is being carried out. Cancer is a disease in which cells grow uncontrollably due to gene mutation, epigenetic abnormality, etc. With regard to genetic abnormalities in cancer, a large number have already been reported (e.g., Futreal et al., Nat Rev Cancer. 2004; 4 (3):177-83, etc.), and it is thought that many thereof are somehow associated with signal transduction related to cell proliferation, differentiation, and survival. Furthermore, due to such genetic abnormalities, abnormalities occur in signal transduction in cells consisting of normal molecules, and this causes activation or inactivation of a specific signal cascade and can finally become one factor triggering abnormal cell proliferation. Early cancer treatment has focused on suppression of cell proliferation itself, but since such a treatment also suppresses proliferation of cells with physiologically normal proliferation, it was accompanied by side effects such as hair loss, gastrointestinal dysfunction, or bone marrow suppression. In order to reduce such side effects, development of drugs for the treatment of cancer based on a new concept such as molecularly targeted drugs that target cancer-specific genetic abnormalities or abnormalities in signal transduction is being undertaken.

As a cancer-specific genetic abnormality, abnormality of KRAS (V-Ki-ras2 Kirsten rat sarcoma viral oncogene homolog) is well known. KRAS is a low molecular weight GTP-binding protein (also called a low molecular weight G protein) positioned downstream of a tyrosine kinase receptor such as EGFR or PDGFR, and is in charge of transferring a signal related to proliferation or differentiation from these receptors to a downstream MAPK cascade. Normal KRAS is activated via Grb2 and SOS by means of tyrosine kinase activation of a receptor activated by ligand binding, and phosphorylates a MAPK such as Raf so as to drive the MAPK cascade, but mutated KRAS is constantly activated without stimulation from a receptor and continues to transmit a proliferation signal. It is thought that because of this, abnormal cell proliferation occurs.

On the one hand, expression of glutathione-S-transferase (GST), which is one of the enzymes that catalyze glutathione conjugation, in particular GST-π (glutathione S-transferase pi, also called GSTP1), increases in various cancer cells, and it has been pointed out that there is a possibility that this is one factor for resistance to some anticancer agents. In fact, it is known that when GST-π antisense DNA or a GST-π inhibitor is made to act on a cancer cell line that is overexpressing GST-π and exhibiting drug resistance, the drug resistance is suppressed (Takahashi and Niitsu, Gan To Kagaku Ryoho. 1994; 21 (7): 945-51, Ban et al., Cancer Res. 1996; 56 (15): 3577-82, Nakajima et al., J Pharmacol Exp Ther. 2003; 306 (3): 861-9). Furthermore, in a recent report, when GST-π siRNA is made to act on an androgen-independent prostate cancer cell line that is overexpressing GST-π, proliferation thereof is suppressed and apoptosis is increased (Hokaiwado et al. , Carcinogenesis. 2008; 29 (6): 1134-8). Moreover, it has been suggested that in human colorectal cancer, KRAS mutation seems to induce overexpression of GST-π via activation of AP-1 (Miyanishi et al., Gastroenterology. 2001; 121 (4): 865-74).

However, there has so far been hardly any clarification of the relationship between GST-π and cell proliferation or apoptosis, the molecular mechanism of GST-π, and the role, etc., of GST-π in various types of intracellular signal transduction. Intracellular signal transduction is very complicated; one molecule may influence the effect of a plurality of molecules, or conversely one molecule may be influenced by a plurality of molecules, when the effect of a certain molecule is inhibited, another signal cascade may be activated, and an expected effect often cannot be obtained. Therefore, it is necessary to elucidate the complicated cell signal transduction mechanism in order to develop better molecularly targeted drugs, but only a very small part of the mechanism has been elucidated in many years of research, and further research effort is needed.

### [Summary]

### [Problems to be Solved]

The current invention is defined, inter alia, by the following items:
1. A composition for the use in the treatment of cancer due to GST-π overexpression and KRAS mutation, wherein the composition comprises as active ingredients a drug that suppresses GST-π and a drug that suppresses autophagy, wherein the drug that suppresses autophagy is a PI3K inhibitor, and wherein the PI3K inhibitor is a class III PI3K inhibitor.
2. The composition according to claim 1 for use according to claim 1, wherein the PI3K inhibitor is 3-methyladenine.
3. The composition according to claim 1 for the use according to claim 1, wherein the PI3K inhibitor is selected from the group consisting of an RNAi molecule, a ribozyme, an antisense nucleic acid, a DNA/RNA chimera polynucleotide, and a vector expressing same.
4. The composition according to any one of the claims above for the use according to any one of the claims above, wherein the drug that suppresses GST-π is selected from the group consisting of an RNAi molecule, a ribozyme, an antisense nucleic acid, a DNA/RNA chimera polynucleotide, and a vector expressing same.

It is an object of the present description to provide novel uses of GST-π and a suppressing agent thereof, a composition for inducing apoptosis effectively in cells, and a method using same.

### [Means for Solving the Problems]

While carrying out intensive research in order to elucidate the molecular mechanism of GST-π, the present inventors have found that, when expression of GST-π is inhibited, activation of Raf-1, MEK, and ERK is greatly inhibited, and similarly in the PI3K (Phosphoinositide 3-kinase) signal cascade, which is activated by activation of a G protein-coupled receptor or a tyrosine kinase receptor, suppression of signal transduction occurs, and have clarified that, although apoptosis is caused by inhibition of GST-π expression, autophagy is induced more rapidly than apoptosis. As a result of further research, it has further been found that by suppressing autophagy at the same time as inhibiting GST-π, cells can be induced to undergo apoptosis with high efficiency, and the present description has thus been accomplished.

That is, the present description relates to the following.
(1) An agent for inducing apoptosis, the agent containing as active ingredients a drug that suppresses GST-π and a drug that suppresses autophagy.
(2) An agent for inducing apoptosis in a cell in which GST-π is suppressed, the agent containing as an active ingredient a drug that suppresses autophagy.
(3) The agent according to (1) or (2) above, the agent being for inducing apoptosis in a cell having mutated KRAS.
(4) The agent according to any one of (1) to (3) above, wherein the active ingredient is selected from the group consisting of an RNAi molecule, a ribozyme, an antisense nucleic acid, a DNA/RNA chimera polynucleotide, and a vector expressing same.
(5) A pharmaceutical composition containing the agent according to any one of (1) to (4) above.
(6) The pharmaceutical composition according to (5) above, the composition being for use in the treatment of a disease caused by abnormal cell proliferation.
(7) The pharmaceutical composition according to (5) above, the composition being for use in the treatment of a disease caused by KRAS mutation.
(8) The pharmaceutical composition according to (5) above, the composition being for use in the treatment of a cancer.
(9) An agent for promoting PI3K/Akt/mTOR signal cascade and/or RAS/Raf/MAPK signal cascade, the agent containing as an active ingredient GST-π and/or a functional variant thereof.
(10) An agent for suppressing PI3K/Akt/mTOR signal cascade and/or RAS/Raf/MAPK signal cascade, the agent containing as an active ingredient a drug that suppresses GST-π.
(11) An agent for suppressing ubiquitination, the agent containing as an active ingredient GST-π and/or a functional variant thereof.
(12) An agent for promoting ubiquitination, the agent containing as an active ingredient a drug that suppresses GST-π.
(13) An agent for suppressing autophagy, the agent containing as an active ingredient GST-π and/or a functional variant thereof.
(14) An agent for promoting autophagy, the agent containing as an active ingredient a drug that suppresses GST-π.

### [Effects]

Since the apoptosis-inducing agent of the present description can induce apoptosis effectively compared with a conventional one, its efficacy as a pharmaceutical composition is also high. In the treatment of cancer in particular, since cancer cells can be killed by apoptosis, not only is it possible to inhibit cancer from progressing, but an effect in making cancer regress can also be expected. Furthermore, since the same level of effect as that of a conventional formulation can be exhibited with a lower dose than that of the conventional one, it becomes possible to reduce side effects.

Moreover, in accordance with the present description, the molecular mechanism of GST-π has become clear and a novel use of GST-π or a suppressing agent thereof has been discovered. This provides new options for the treatment of disease, experimental techniques, etc., and an enormous contribution can be expected not only to medicine and veterinary medicine but also to the fields of biology, biochemistry, molecular biology, etc.

### [Brief Description of Drawings]

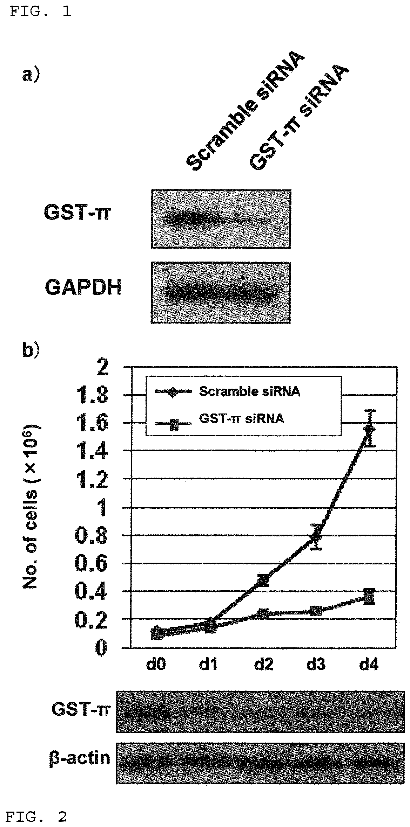

FIG. 1 a) is the result of western blotting showing a state in which expression of GST-n is specifically suppressed by GST-π siRNA. FIG. 1 b) is a diagram showing change in the number of cells and expression level of GST-π on the 1st to 4th day after GST-π siRNA transfection.
FIG. 2 is the result of western blotting showing the state of expression of protein involved in the RAS/Raf/MAPK signal cascade on the 2nd day after GST-π siRNA transfection. It can be seen that, accompanying suppression of GST-π expression, the expression level of Raf protein decreases and, furthermore, phosphorylation of a MAPK such as MEK or ERK is suppressed.

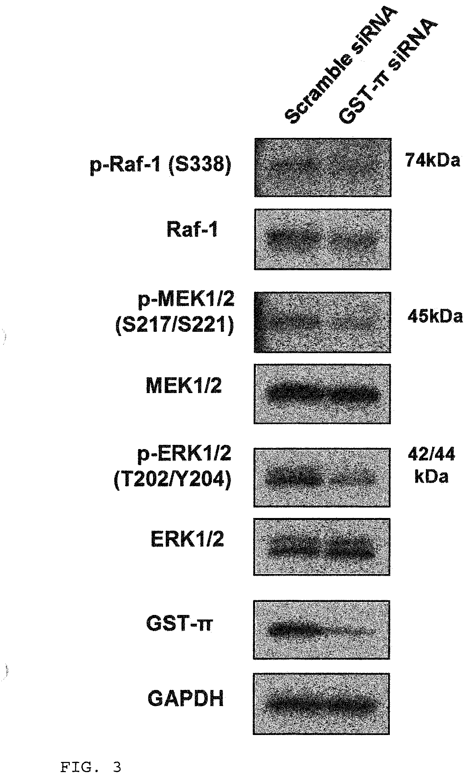

FIG. 3 shows the result of an experiment on the immunoprecipitation of Raf protein. It can be seen that in a GST-π siRNA treated group expression of Raf protein and Ser621 phosphorylated Raf protein (p-Raf-1 (S621)) decreased slightly, whereas in contrast ubiquitinated Raf protein increased.

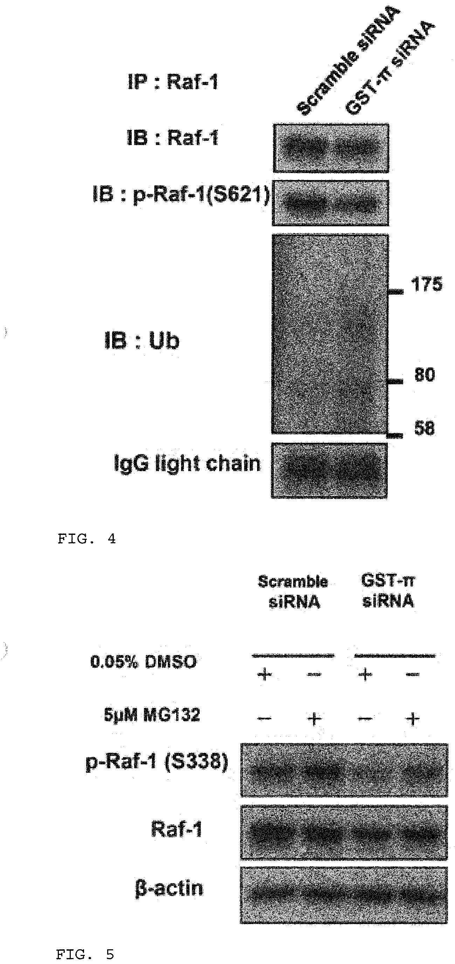

FIG. 4 is a diagram comparing the abundance of Raf protein when GST-π siRNA transfectant and Scramble siRNA transfectant were treated with proteasome inhibitor MG132 and DMSO, which was a negative control. It was observed that for the GST-π siRNA transfectant, treating with proteasome inhibitor increased the abundance of phosphorylated Raf protein (p-Raf-1 (S338)), but no change was observed for Scramble siRNA. That is, this suggests that due to suppression of GST-π expression, phosphorylated Raf protein undergoes degradation by proteasome, and this is consistent with the increase in ubiquitinated Raf protein in FIG. 3.
FIG. 5 shows the results of an experiment on the co-immunoprecipitation of Raf protein and GST-π. Because of a reaction toward anti-GST-π antibody being shown for protein precipitated by anti-p-Raf-1 antibody, it is suggested that p-Raf-1 and GST-π form a complex.

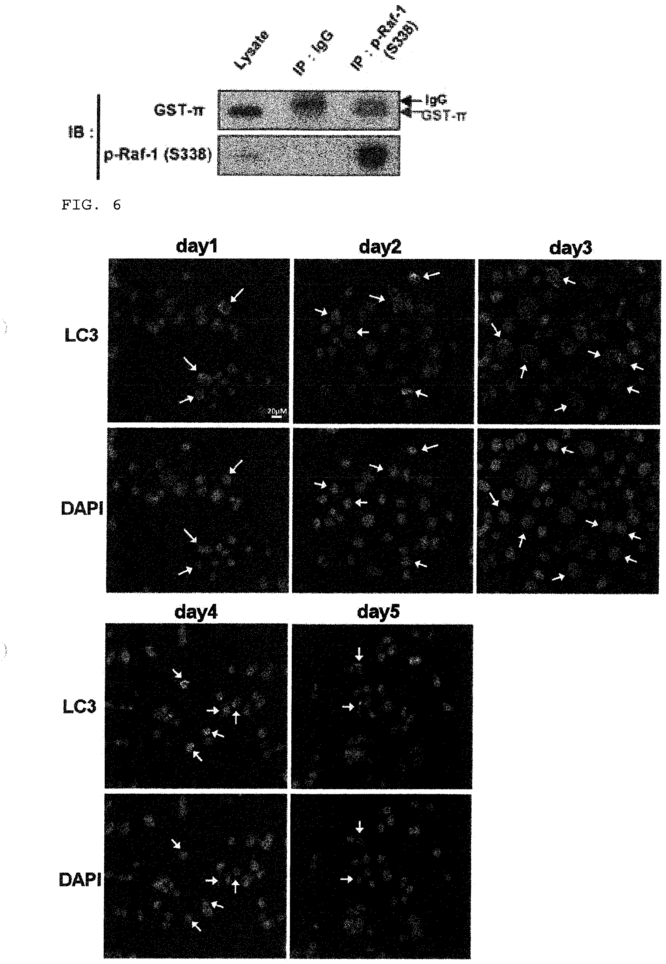

FIG. 6 shows immunofluorescence staining images of GST-π knockdown cells. The upper rows are anti-LC3 antibody stained images, and the lower rows are DAPI stained images. From the left on the top are stained images of the 1st day, 2nd day, and 3rd day after transfection, and from the left on the bottom of the 4th day and 5th day after transfection, respectively. In cells marked by arrows in the figure, a spot signal that can be considered to be an autophagosome was observed, inferring that autophagy is induced.

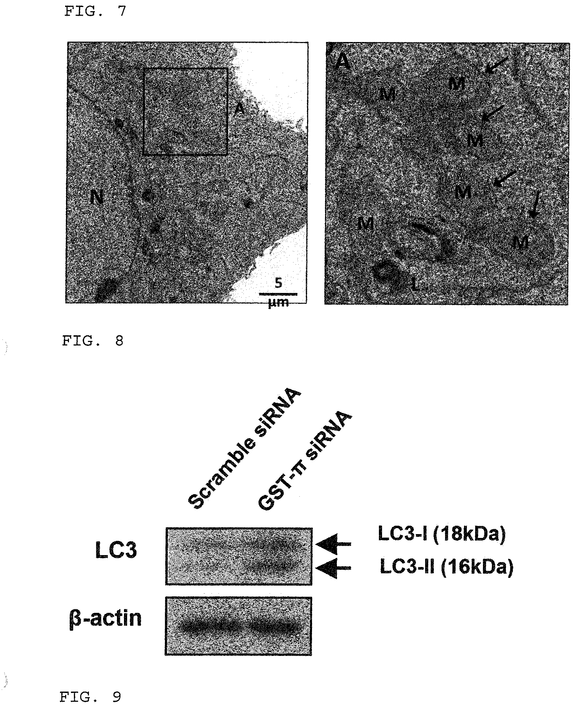

FIG. 7 shows electron microscopy images of GST-π knockdown cells at the point in time when 2 days had elapsed after GST-π siRNA transfection. In the left-hand figure, N denotes the nucleus, and the right-hand figure is an enlarged image of part A surrounded by the square. In the right-hand figure, M denotes mitochondria and L denotes lysosome. It was observed that autophagosomes were formed so as to surround mitochondria in the areas shown by arrows.
FIG. 8 shows the results of western blotting of a GST-π knockdown (KD) cell extract using anti-LC3 antibody. It was observed that in GST-π KD cells (GST-π siRNA), expression of LC3 markedly increased compared with the control (Scramble siRNA). Since type II LC3 (LC3-II) in particular increased greatly, this infers the induction of autophagosome.
FIG. 9 shows the results of TUNEL staining. The upper row shows images of a control group and the lower row shows images of a GST-π KD cell group. It shows from the left stained images of the 3rd day, 4th day, and 5th day after transfection. In the GST-π KD cell group, TUNEL-positive cells were observed.

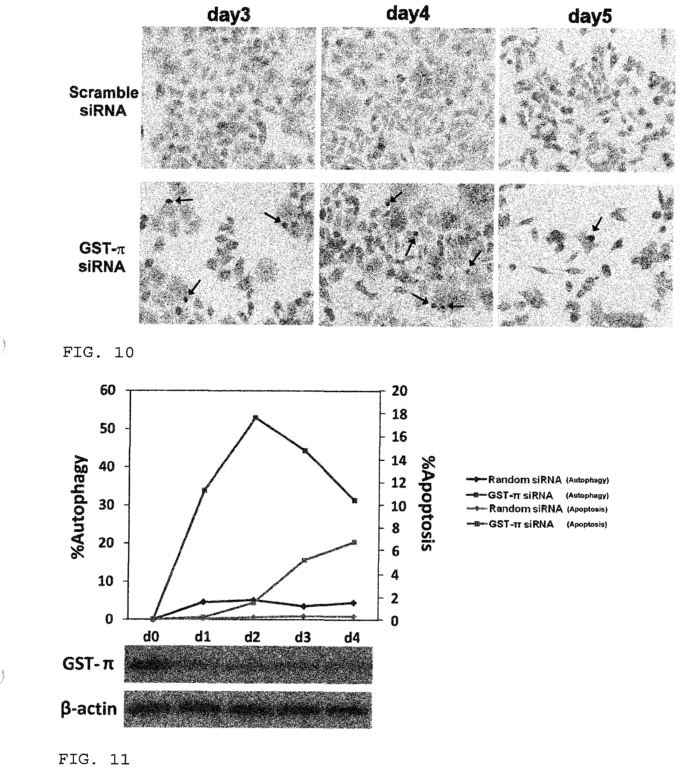

FIG. 10 is a graph (top) showing change over time of the proportions of autophagy-positive cells and apoptosis-positive cells in a GST-π KD cell group and a control cell group at the point in time when 1 to 4 days had elapsed after siRNA transfection, and a diagram (bottom) showing the expression level of GST-π in GST-π KD cells. It shows that in the control group hardly any autophagy or apoptosis was observed, whereas in the GST-π KD group autophagy-positive cells first rapidly increased with a peak on the 2nd day, and then apoptosis was induced.
FIG. 11 shows the results of western blotting of protein involved in EGFR/PI3K/Akt/mTOR signaling in GST-π KD cells. It can be seen that in a GST-π KD cell group, phosphorylation of EGFR, PI3K and Akt was markedly suppressed.

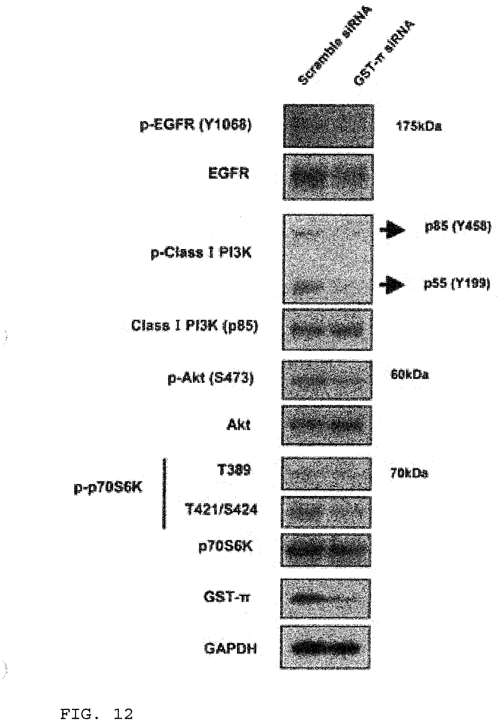

FIG. 12 shows the results of western blotting showing change in expression of phosphorylated EGFR (p-EGFR) when GST-π KD cells were treated with proteasome inhibitor MG132. Since a decrease in the expression level of p-EGFR in GST-π KD cells was recovered by treatment with proteasome inhibitor, it was inferred that the decrease in the expression of p-EGFR was due to degradation by proteasome.

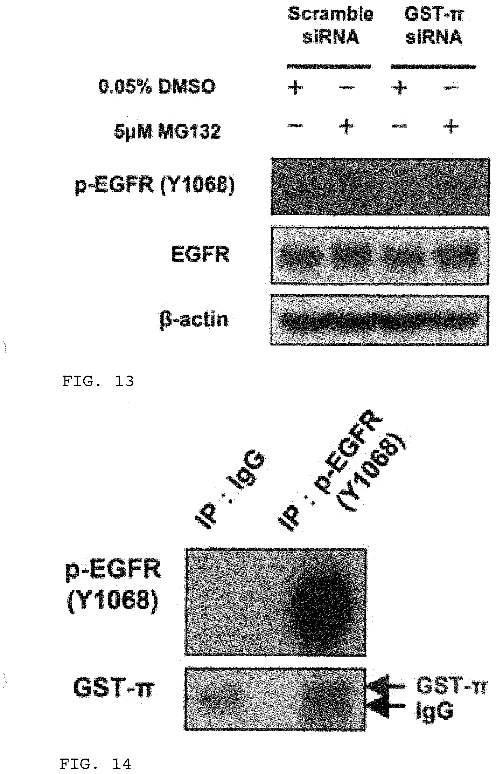

FIG. 13 shows the result of western blotting of protein co-immunoprecipitated using anti-p-EGFR. Since a signal was observed for anti-GST-n antibody, it was inferred that p-EGFR and GST-π interacted with each other.
FIG. 14 shows the results when examining the change of the expression level of Raf protein and EGFR depending on inhibitor concentration when the GST-π inhibitor C16C2 was used. It was observed that, as in the case with GST-π knockdown, phosphorylation of EGFR or Raf protein was also suppressed when the GST-π inhibitor was used.

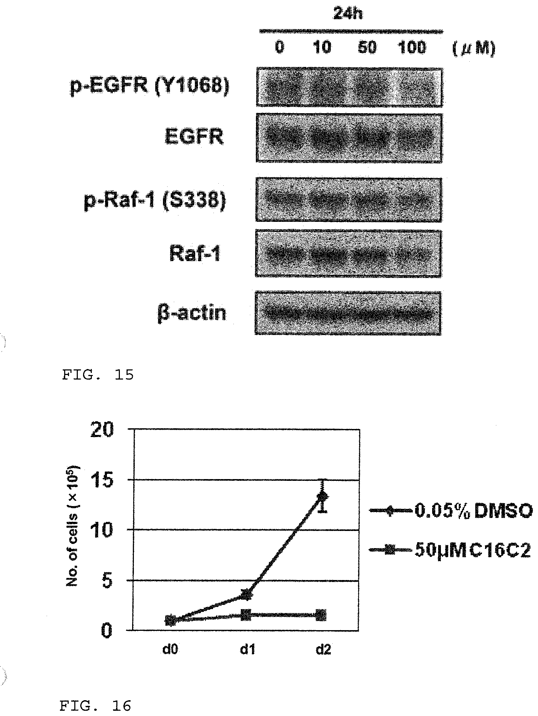

FIG. 15 is a graph showing change in the number of cells when the GST-π inhibitor C16C2 was added. It can be seen that when the GST-π inhibitor was added, there was hardly any increase in the number of cells.
FIG. 16 is a graph showing the proportion of autophagy-positive cells in a Scramble siRNA treated group, a GST-π KD group, and a GST-π KD + 3MA group. It can be seen that the autophagy that had been increased by knockdown of GST-π was suppressed by 3-MA.

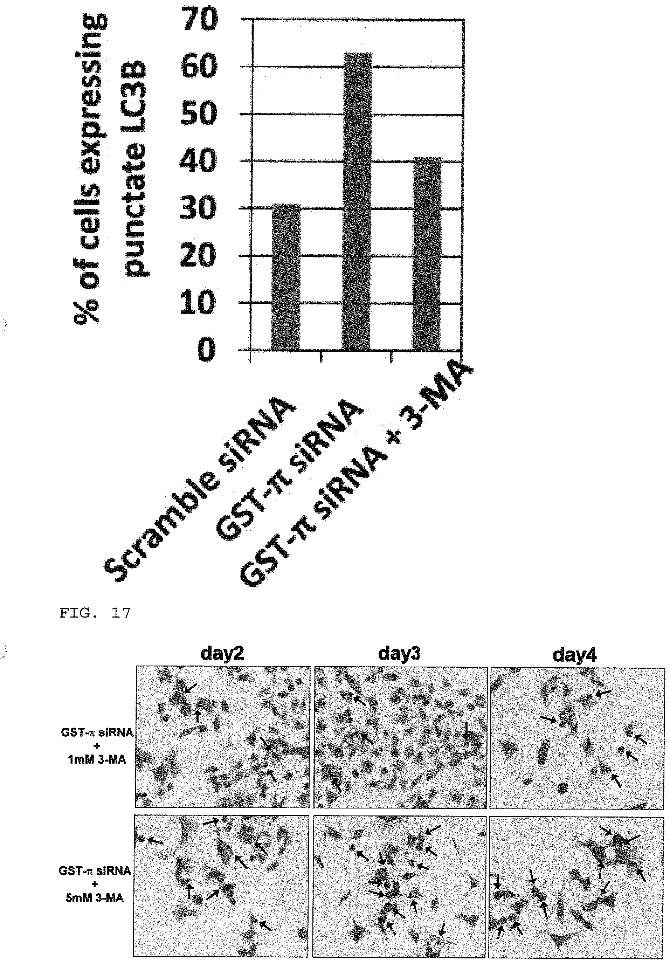

FIG. 17 shows TUNEL stained images when 3-MA was added to GST-π KD cells. The upper row is a case where 3-MA was added at 1 mM, and the lower row is a case where 3-MA was added at 5 mM. It shows from the left stained images on the 2nd day, 3rd day, and 4th day after transfection. The larger the amount of 3-MA added, the more apoptotic cells were observed.

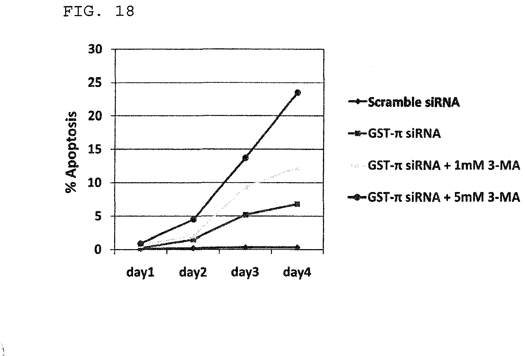

FIG. 18 is a graph showing the results when percentage apoptosis was examined over time in a control cell group (Scramble siRNA), a GST-π KD cell group (GST-π siRNA), a GST-π KD cell + 1 mM 3-MA group (GST-π siRNA + 1 mM 3-MA), and a GST-π KD cell + 5 mM 3-MA group (GST-π siRNA + 5 mM 3-MA). It was found that there was a further 3-MA dose-dependent induction of apoptosis.

### [Modes for Carrying Out the Invention]

The present description relates to an agent or composition for inducing apoptosis (hereinafter, also called an 'apoptosis-inducing agent' or an 'apoptosis-inducing composition') that contains as active ingredients a drug that suppresses GST-π and a drug that suppresses autophagy.

When used herein, GST-π denotes an enzyme, encoded by GSTP1 gene, that catalyzes glutathione conjugation. GST-π is present in various animals, including humans, and its sequence information is known (e.g., human: NP_000843 (NM_000852), rat: NP_036709 (NM_012577), mouse: NP_038569 (NM_013541), etc. The numbers denote NCBI database accession numbers; those outside parentheses are amino acid sequence numbers, and those inside parentheses are base sequence numbers).

Since there is a possibility of the occurrence of a mutation of a gene sequence or an amino acid sequence between biological individuals that does not impair the physiological function of a protein, GST-π and GSTP1 gene in the present invention are not limited to a protein or nucleic acid having the same sequence as the above known sequences, and can include those that have a sequence that is different from the above sequence by one or more amino acids or bases, typically one or a few, for example, one, two, three, four, five, six, seven, eight, nine, or ten amino acids or bases, but have an equivalent function to that of the known GST-π. The specific function of GST-π is as described later.

In the present specification, phrases such as 'when used herein', 'used herein', 'in the present specification', and 'described herein' mean, unless otherwise specified, that the description following them applies to all of the description described in the present specification. Furthermore, unless otherwise defined, all of the technical terms and scientific terms used herein have the same meaning as that usually understood by a person skilled in the art.

Examples of the 'drug that suppresses GST-π' used herein include, but are not limited to, a drug that suppresses GST-π production and/or activity and a drug that promotes GST-π degradation and/or inactivation. Examples of the drug that suppresses GST-π production include, but are not limited to, an RNAi molecule, ribozyme, antisense nucleic acid, or DNA/RNA chimera polynucleotide for DNA encoding GST-π, or a vector expressing same.

Examples of the drug that suppresses GST-π activity include, but are not limited to, a substance that binds to GST-π such as, for example, glutathione, a glutathione analog (e.g., those described in WO 95/08563, WO 96/40205, WO 99/54346, Nakajima et al., 2003, supra, etc.), ketoprofen (Takahashi and Niitsu, 1994 supra), indomethacin (Hall et al., Cancer Res. 1989; 49 (22): 6265-8), ethacrynic acid, Piloprost (Tew et al., Cancer Res. 1988; 48 (13): 3622-5), an anti-GST-π antibody, and a GST-π dominant negative mutant. These drugs are either commercially available or may be produced appropriately based on known techniques.

The drug that suppresses GST-π production or activity is preferably an RNAi molecule, ribozyme, antisense nucleic acid, or DNA/RNA chimera polynucleotide for DNA encoding GST-π, or a vector expressing same, in terms of high specificity and a low possibility of side effects.

Suppression of GST-π may be determined by the expression or activity of GST-π in cells being suppressed compared with a case in which a GST-π suppressing agent is not utilized. Expression of GST-π may be evaluated by any known technique; examples thereof include, but are not limited to, an immunoprecipitation method utilizing an anti-GST-π antibody, EIA, ELISA, IRA, IRMA, a western blot method, an immunohistochemical method, an immunocytochemical method, a flow cytometry method, various hybridization methods utilizing a nucleic acid that specifically hybridizes with a nucleic acid encoding GST-π or a unique fragment thereof, or a transcription product (e.g., mRNA) or splicing product of said nucleic acid, a northern blot method, a Southern blot method, and various PCR methods.

Furthermore, the activity of GST-π may be evaluated by analyzing a known activity of GST-π including, but not limited to, binding to a protein such as, for example, Raf-1 (in particular phosphorylated Raf-1) or EGFR (in particular phosphorylated EGFR) by means of any known method such as for example an immunoprecipitation method, a western blot method, a mass analysis method, a pull-down method, or a surface plasmon resonance (SPR) method.

When used herein, the RNAi molecule denotes any molecule that causes RNA interference, including, but not limited to, a duplex RNA such as siRNA (small interfering RNA), miRNA (micro RNA), shRNA (short hairpin RNA), ddRNA (DNA-directed RNA), piRNA (Piwi-interacting RNA), or rasiRNA (repeat associated siRNA) and modified forms thereof. These RNAi molecules may be commercially available or may be designed and prepared based on known sequence information, etc.

Furthermore, when used herein, the antisense nucleic acid includes RNA, DNA, PNA, or a complex thereof.

When used herein, the DNA/RNA chimera polynucleotide includes, but is not limited to, a double-strand polynucleotide composed of DNA and RNA that inhibits the expression of a target gene described in for example JP, A, 2003-219893.

When used herein, autophagy can include macroautophagy, microautophagy, chaperone-mediated autophagy, etc., but typically means macroautophagy. Therefore, the term 'autophagy' in the present invention refers to 'macroautophagy' unless otherwise specified.

Autophagy, meaning 'self-devouring', is one of the intracellular protein degradation mechanisms, and is in charge of the degradation and recycling of protein within a cell. Autophagy is seen in a wide variety of biological species including yeasts and mammals and is generally accompanied by a series of processes including (a) formation of a PAS (phagophore assembly site), (b) elongation and extension of the phagophore surrounding a protein to be degraded (isolation membrane) and formation of an autophagosome encapsulating the protein to be degraded, (c) formation of an autolysosome by fusion of an autophagosome and a lysosome, and (d) degradation of the protein within the autolysosome.

The above processes (a) to (c) involve specific autophagy-related factors. With regard to the autophagy-related factors, the first research was carried out with yeast, and a large number, including ATG1 to ATG27, have been identified so far (Klionsky et al., Dev Cell. 2003; 5 (4): 539-45); research with mammals has also advanced, a plurality of homologs have been identified, and the core molecular mechanism of autophagy is becoming clear (Yang and Klionsky, Curr Opin Cell Biol. 2010; 22 (2): 124-31).

Examples of autophagy-related factors involved in the core molecular mechanism of autophagy in mammals include those involved in formation of PAS, such as VMP1, TP53INP2, mAtg9, the ULK complex (composed of ULK1, ULK2, mAtg13, and FIP200), the PI3K complex (the Atg14L complex composed of Beclin1, hVps34, p150, Ambra1, and Atg14L, and the UVRAG complex composed of Beclin1, hVps34, p150, Bif-1, and UVRAG) and those involved in phagophore elongation such as LC3-II and the Atg12-Atg5-Atg16L complex.

Therefore, examples of the drug that suppresses autophagy include, but are not limited to, a drug that suppresses the production and/or activity of an autophagy-related factor such as those described above and a drug for promoting the degradation and/or inactivation of an autophagy-related factor. Examples of the drug that suppresses the production of an autophagy-related factor include an RNAi molecule, ribozyme, antisense nucleic acid, or DNA/RNA chimera polynucleotide for DNA encoding an autophagy-related factor, or a vector expressing same.

Examples of the drug that suppresses the activity of an autophagy-related factor include, but are not limited to, a PI3K inhibitor (e.g., wortmannin, etc.), in particular a class III PI3K inhibitor (e.g., 3-MA (3-methyladenine), etc.), a substance that inhibits fusion of an autophagosome and a lysosome (e.g., bafilomycin A1, etc.), a substance that inhibits protein degradation in an autolysosome (e.g., chloroquine, leupeptin, etc.), a substance that binds to an autophagy-related factor (e.g., an antibody for an autophagy-related factor, etc.), and a dominant negative mutant of an autophagy-related factor. These drugs are commercially available or may be produced appropriately based on known techniques. In one embodiment of the present description, the drug that suppresses autophagy does not contain GST-π and/or a functional variant thereof.

From the viewpoint of high specificity and low side effects, the drug that suppresses autophagy is preferably an RNAi molecule, ribozyme, antisense nucleic acid, or DNA/RNA chimera polynucleotide for DNA encoding an autophagy-related factor, or a vector expressing same.

Suppression of autophagy may be determined by observing that autophagy is suppressed in cells compared with a case in which the autophagy suppressing agent of the present description is not utilized. Inhibition of autophagy may be evaluated based on any known technique, examples of which include, but not limited to, detection of an autophagosome by an electron microscopy method, and detection of an autophagy marker (e.g., Atg5, Atg12, LC3, in particular LC3-II, etc.). LC3-II may be detected, for example, but not limited to, by using a specific antibody for LC3-II, or may be detected by subjecting a sample to separation with electrophoresis, etc., and then detecting LC3-II, separated as a band that is different from LC3-I, by a western blot method, etc. , using an antibody that reacts with LC3-II or both LC3-I and LC3-II. Furthermore, because LC3-I is dispersed within the cytoplasm, while LC3-II is localized in an autophagy-specific structure such as an isolation membrane, an autophagosome, or an autolysosome, the presence or number of spot-like signals showing these structures, which are manifested by immunostaining, etc., with an antibody that reacts with LC3-II (including an antibody that reacts to both LC3-I and LC3-II) may be used as an indicator for autophagy.

The drug that suppresses GST-π and the drug that suppresses autophagy may be contained in a single formulation or may be contained separately in two or more formulations. In the case of the latter, each formulation may be administered at the same time or they may be administered with a time interval therebetween. When administered with a time interval therebetween, the formulation containing a drug that suppresses GST-π may be administered prior to the formulation containing a drug that suppresses autophagy or may be administered subsequent thereto.

The present description also relates to an agent or composition for inducing apoptosis (hereinafter, also called an 'apoptosis-inducing agent' or an 'apoptosis-inducing composition') in cells in which GST-π is suppressed, the agent or composition containing as an active ingredient a drug that suppresses autophagy.

When used herein, 'GST-π being suppressed' includes for example a state in which GST-π is being suppressed in cells expressing GST-π. Examples of such a state include a state in which a drug that suppresses GST-π (e.g., those described above, etc.) has been administered to cells expressing GST-π.

Whether or not GST-π is being expressed in certain cells is either known from the literature or may be determined by actually detecting expression of GST-π in cells. Expression of GST-π may be detected by any known technique, including those described above.

The agent or composition of the present description may be one for inducing apoptosis in cells having a mutated KRAS.

When used herein, examples of the mutated KRAS include, but are not limited to, those having a mutation that causes constant activation of KRAS, such as a mutation that inhibits endogenous GTPase or a mutation that increases the guanine nucleotide exchange rate. Specific examples of such mutation include, but are not limited to, for example, mutation in amino acids 12, 13 and/or 61 in human KRAS (inhibiting endogenous GTPase) and mutation in amino acids 116 and/or 119 in human KRAS (increasing guanine nucleotide exchange rate) (Bos, Cancer Res. 1989; 49 (17): 4682-9, Levi et al., Cancer Res. 1991; 51 (13): 3497-502). Therefore, in one embodiment of the present invention, the mutated KRAS can be a KRAS having a mutation at at least one of amino acids 12, 13, 61, 116, and 119 of human KRAS. In one embodiment of the present invention, the mutated KRAS has a mutation at amino acid 12 of human KRAS. Furthermore, in one embodiment of the present invention, the mutated KRAS may be one that induces overexpression of GST-π. Therefore, cells having mutated KRAS may exhibit overexpression of GST-π.

Detection of mutated KRAS may be carried out using any known technique. Examples of such a technique include, but are not limited to, selective hybridization by means of a nucleic acid probe specific to a known mutation sequence, an enzyme mismatch cleavage method, sequencing (Bos, 1989 supra), and a PCR-RFLP method (Miyanishi et al., 2001 supra).

Furthermore, detection of GST-π expression may be carried out using any known technique, including those described above. Whether or not GST-π is being overexpressed may be evaluated by for example comparing the degree of expression of GST-π in cells having mutated KRAS with the degree of expression of GST-π in the same type of cells having normal KRAS. In this case, it can be said that GST-π is being overexpressed if the degree of expression of GST-π in cells having mutated KRAS exceeds the degree of expression of GST-π in the same type of cells having normal KRAS.

The amount of active ingredient formulated in the agent or composition of the present description may be an amount that induces apoptosis when the agent or composition is administered. Furthermore, it is preferably an amount that does not cause an adverse effect that exceeds the benefit of administration. Such an amount is known or may be determined appropriately by an in vitro test using cultured cells, etc., or a test in a model animal such as a mouse, a rat, a dog, or a pig, and such test methods are well known to a person skilled in the art. Induction of apoptosis may be evaluated by various known techniques, for example, by detection of an apoptosis-specific phenomenon such as DNA fragmentation, binding of annexin V to cell membrane, change in mitochondrial membrane potential, or activation of caspase, or by TUNEL staining. The amount of active ingredient formulated can vary according to the manner in which the agent or composition is administered. For example, when a plurality of units of the composition is used for one administration, the amount of active ingredient to be formulated in one unit of the composition may be determined by dividing the amount of active ingredient necessary for one administration by said plurality of units. Adjustment of such a formulation amount can be carried out appropriately by a person skilled in the art.

The present description also relates to a process for producing an agent or composition for inducing apoptosis, the process comprising formulating as active ingredients a drug that suppresses GST-π and a drug that suppresses autophagy; use of a drug that suppresses GST-π and a drug that suppresses autophagy in the production of an agent or composition for inducing apoptosis; a combination of a drug that suppresses GST-π and a drug that suppresses autophagy for use in the induction of apoptosis; and a method of inducing apoptosis comprising administering effective amounts of drug that suppresses GST-π and drug that suppresses autophagy.

The present description also relates to a process for producing an agent or composition for inducing apoptosis in cells in which GST-π is suppressed, the process comprising formulating as an active ingredient a drug that suppresses autophagy; use of a drug that suppresses autophagy in the production of an agent or composition for inducing apoptosis in cells in which GST-π is suppressed; a drug that suppresses autophagy for use in the induction of apoptosis in cells in which GST-π is suppressed; and a method of inducing apoptosis in cells in which GST-π is suppressed, the method comprising administering an effective amount of a drug that suppresses autophagy.

The drug or the formulation amount thereof in the above-mentioned production process or use are as described above. Formulation of each drug may be carried out in accordance with any known technique.

All of the above methods for inducing apoptosis may be either an in vitro method or an in vivo method. Furthermore, the drugs in the methods are as described above, and the effective amount of drug may be an amount that induces apoptosis in cells to which it is administered. It is also preferably an amount that does not cause an adverse effect that exceeds the benefit of administration. Such an amount is known or may be determined appropriately by an in vitro test using cultured cells, etc., and such a test method is well known to a person skilled in the art. Induction of apoptosis may be evaluated by various known techniques, including those described above. The effective amount above need not necessarily be one that induces apoptosis in all the cells of a cell population to which the drug is administered. For example, the effective amount above may be an amount that induces apoptosis in, of the cell population, at least 1% of the cells, at least 2%, at least 3%, at least 4%, at least 5%, at least 6%, at least 8%, at least 10%, at least 12%, at least 15%, at least 20%, at least 25%, etc.

The apoptosis-inducing agent of the present description can induce apoptosis effectively even in cells having an abnormality in cell proliferation, etc., and is effective as a component of a pharmaceutical composition. Therefore, one aspect of the present description includes a pharmaceutical composition containing the apoptosis-inducing agent of the present description .

The pharmaceutical composition of the present description is effective in treating a disease in which there is abnormal apoptosis in particular. Therefore, one embodiment of the present description relates to a pharmaceutical composition for treating a disease in which there is abnormal apoptosis, the pharmaceutical composition containing the apoptosis-inducing agent. When used herein, examples of the disease in which there is abnormal apoptosis include, but are not limited to, a disease due to abnormal cell proliferation, a disease due to KRAS mutation, and a disease due to GST-π overexpression. Examples of the disease due to abnormal cell proliferation include, but are not limited to, a benign or malignant tumor, hyperplasia, keloid, Cushing's syndrome, primary aldosteronism, erythroplakia, polycythemia vera, leukoplakia, hyperplastic scar, lichen planus, and lentiginosis. Examples of the disease due to KRAS mutation include, but are not limited to, a benign or malignant tumor (also called a cancer or a malignant neoplasm). Examples of the disease due to GST-π overexpression include, but are not limited to, a benign or malignant tumor, in particular a drug-resistant malignant tumor (e.g., resistant to an alkylating agent such as melphalan or cyclophosphamide, an anthracycline-based antitumor antibiotic such as adriamycin, a platinum complex such as cisplatin, etoposide, etc.). In one embodiment of the present description, the disease in which there is abnormal apoptosis is a cancer.

Examples of the cancer in the present description include, but are not limited to, sarcomas such as fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, and osteosarcoma, carcinomas such as brain tumor, head and neck carcinoma, breast carcinoma, lung carcinoma, esophageal carcinoma, gastric carcinoma, duodenal carcinoma, appendiceal carcinoma, colon carcinoma, rectal carcinoma, liver carcinoma, pancreatic carcinoma, gall bladder carcinoma, bile duct carcinoma, anal carcinoma, renal carcinoma, ureteral carcinoma, bladder carcinoma, prostate carcinoma, penile carcinoma, testicular carcinoma, uterine carcinoma, ovarian carcinoma, vulvar carcinoma, vaginal carcinoma, and skin carcinoma and, furthermore, leukemia and malignant lymphoma. In the present invention, 'cancer' includes epithelial malignancy and non-epithelial malignancy. The cancer in the present description can be present at any site of the body, for example, the brain, head and neck, chest, limbs, lung, heart, thymus, esophagus, stomach, small intestine (duodenum, jejunum, ileum), large intestine (colon, cecum, appendix, rectum), liver, pancreas, gallbladder, anus, kidney, urinary duct, bladder, prostate, penis, testis, uterus, ovary, vulva, vagina, skin, striated muscle, smooth muscle, synovial membrane, cartilage, bone, thyroid, adrenal gland, peritoneum, mesentery, bone marrow, blood, vascular system, lymphatic system such as lymph node, lymphatic fluid, etc.

In one embodiment of the present description, the cancer includes cancer cells having the mutated KRAS defined above. In one embodiment of the present description, the cancer includes cancer cells that exhibit hormone- or growth factor-independent proliferation. In one embodiment of the present description, the cancer includes cancer cells exhibiting GST-π overexpression. In one embodiment of the present description, the cancer is drug resistant. In one embodiment of the present description, the cancer has resistance to a drug selected from the group consisting of an alkylating agent such as melphalan or cyclophosphamide, an anthracycline-based antitumor antibiotic such as adriamycin, a platinum complex such as cisplatin, and etoposide. In one embodiment of the present description, the cancer has resistance to a medicinal agent selected from the group consisting of melphalan, cyclophosphamide, adriamycin, cisplatin, and etoposide.

The present description also relates to a pharmaceutical composition for treating a disease in which there is abnormal apoptosis, the composition containing as active ingredients a drug that suppresses GST-π and a drug that suppresses autophagy; a process for producing a pharmaceutical composition for treating a disease in which there is abnormal apoptosis, the process comprising formulating as active ingredients a drug that suppresses GST-π and a drug that suppresses autophagy; use of a drug that suppresses GST-π and a drug that suppresses autophagy for the production of a pharmaceutical composition for treating a disease in which there is abnormal apoptosis; a combination of a drug that suppresses GST-π and a drug that suppresses autophagy for use in the treatment of a disease in which there is abnormal apoptosis; and a method for treating a disease in which there is abnormal apoptosis, the method comprising administering an effective amount of the pharmaceutical composition to a subject that requires same.

The drug, the formulation amount, and the disease in which there is abnormal apoptosis in the production process or use are as described above. Formulation of each drug may be carried out in accordance with any known technique.

The present inventors have now clarified that GST-π binds to a tyrosine kinase receptor, which is on the upstream of the PI3K/Akt/mTOR signal cascade, in particular to its phosphorylated form, and to Raf, which is a constituent molecule of the RAS/Raf/MAPK signal cascade, in particular to its phosphorylated form, to thus inhibit ubiquitination of these molecules, thereby promoting these signal cascades. Therefore, the present description also relates to an agent or composition for promoting the PI3K/Akt/mTOR signal cascade and/or the RAS/Raf/MAPK signal cascade (also called a 'signal cascade promoter' or a 'signal cascade promoting composition'), the agent or composition containing as an active ingredient GST-π and/or a functional variant thereof. In particular, the agent or composition of the present description can promote both the PI3K/Akt/mTOR signal cascade and the RAS/Raf/MAPK signal cascade at the same time.

Examples of the 'GST-π functional variant' used herein include, but are not limited to, (i) a variant that has one or more mutations, typically one or a few, in the GST-π amino acid sequence but still has an equivalent function to GST-π, (ii) a variant that is encoded by a nucleic acid having a base sequence of a gene encoding GST-π or a nucleic acid having one or more mutations, typically one or a few, in the base sequence of a nucleic acid encoding the same polypeptide as that encoded by the above nucleic acid, and has an equivalent function to GST-π, (iii) a variant that is encoded by a nucleic acid that hybridizes, under stringent conditions, to a complementary strand of a nucleic acid having a base sequence of a gene encoding GST-π, a nucleic acid encoding the same polypeptide as that encoded by the above nucleic acid, or a nucleic acid encoding a variant of (ii), or a fragment of the complementary strand, and has an equivalent function to GST-π, (iv) a variant that has an amino acid sequence having a homology of at least 60%, preferably at least 70%, more preferably at least 80%, yet more preferably at least 90%, and particularly preferably at least 95% with the amino acid sequence of GST-π, and has an equivalent function to GST-π, and (v) a variant that is encoded by a nucleic acid having a homology of at least 60%, preferably at least 70%, more preferably at least 80%, yet more preferably at least 90%, and particularly preferably at least 95% with the base sequence of the gene encoding GST-π, and has an equivalent function to GST-π.

The amino acid sequence of GST-π and the base sequence of the gene encoding GST-π are known for various type of animals as described above, and a person skilled in the art can appropriately prepare the above-mentioned functional variants based on these sequence information by any known technique, for example, chemical synthesis, cleavage or insertion of a nucleic acid by a restriction enzyme, site-directed mutagenesis, application of radiation or UV rays, etc.

Whether or not a given variant has an equivalent function to GST-π may be evaluated by analyzing a known function of GST-π including, for example, but not limited to, binding with a protein such as Raf-1 (in particular phosphorylated Raf-1) or EGFR (in particular phosphorylated EGFR) by any known method such as for example an immunoprecipitation method, a western blot method, a mass analysis method, a pull-down method, or a surface plasmon resonance (SPR) method, and comparing it with an appropriate negative control or GST-π as a positive control. For example, when the function of a given variant is superior to a negative control, for example when it is superior by at least 10%, at least 25%, at least 50%, at least 75%, or at least 100% and/or when the function is at least 1/100 of GST-π, at least 1/50, at least 1/25, at least 1/10, at least 1/5, or at least 1/2, this variant is included in the functional variants of GST-π.

The term 'stringent conditions' used herein is a known parameter in this technical field and is described in a standard protocol such as for example Sambrook et al., Molecular Cloning: A Laboratory Manual, 3d ed., Cold Spring Harbor Press (2001) or Ausubel et al., Current Protocols in Molecular Biology, Greene Publishing Associates (1992).

The stringent conditions in the present description mean for example hybridization at 65°C by means of a hybridization buffer containing 3.5× SSC (0.15 M sodium chloride/0.15 M sodium citrate, pH7), Ficoll 0.02%, polyvinylpyrrolidone 0.02%, bovine serum albumin 0.02%, NaH₂PO₄ 25 mM (pH7), SDS 0.05%, and EDTA 2 mM. After hybridization, a membrane to which DNA has been transferred is washed with 2× SSC at room temperature, and then with 0.1 to 0.5× SSC/0.1× SDS at a temperature up to 68°C. Alternatively, the stringent hybridization may be carried out using a commercial hybridization buffer such as ExpressHyb^{(R)} Hybridization Solution (Clontech) under hybridization and washing conditions described by the manufacturer.

There are other applicable conditions, reagents, etc., that can result in the same degree of stringency, but since a person skilled in the art can be expected to be familiar with such conditions, they are not specifically referred to herein. However, it is possible to manipulate conditions in order to clearly identify a nucleic acid encoding a GST-π variant.

GST-π and/or a functional variant thereof in the present description include, in addition to GST-π and a functional variant thereof as proteins, a nucleic acid encoding GST-π and a nucleic acid encoding a functional variant of GST-π.

When used herein, the 'signal cascade' means signal transduction via which a plurality of signaling molecules transmit a signal in sequence. For example, in the case of the 'PI3K/Akt/mTOR signal cascade', a signal is transmitted in such a manner that PI3K is first activated, this then causes Akt to be activated, and this then causes mTOR to be activated. This also applies in the notation of other signal cascades. With regard to the relationship between upstream and downstream of signaling, the chain of activation may be caused either directly or indirectly. For example, it is known that activation of Akt caused by PI3K is mediated by molecules such as PIP₃ (Phosphatidylinositol (3,4,5) trisphosphate) and PDK1 (Phosphoinositide dependent protein kinase 1, also called PDPK1).

The PI3K/Akt/mTOR signal cascade is a signal cascade that is driven by activation of PI3K and is known to be signaling involved in cell survival, etc. Examples of the way in which activation of PI3K occurs include, but are not limited to, a ligand binding to a G protein-coupled receptor or a tyrosine kinase receptor, and activated PI3K phosphorylates inositol phospholipid, thus producing a phosphatidylinositol such as PIP₃. This binds to PDK1 or Akt at a PH domain, thus promoting localization of these proteins in the membrane. PDK1 binds to PIP3 to thus be activated in the membrane, and the activated PDK1 phosphorylates T at the 308^{th} position of Akt. Serine at the 473^{rd} position of Akt is phosphorylated by mTORC2, which is one of the mTOR complexes, and Akt is completely activated as a result of phosphorylation of the amino acids at these two positions.

Although the route for activation of mTOR by Akt has not been completely elucidated, it is thought that PRAS40 (proline-rich Akt/PKB substrate 40 kDa) is involved. PRAS40 is an Akt substrate as the name indicates and is a molecule that is thought to bind to an mTOR complex to thus suppress its activation, and it is thought that when Akt is activated PRAS40 is phosphorylated, thereby causing PRAS40 to be released from the mTOR complex to thus activate mTOR. When mTOR is activated, ULK1 and ULK2 (unc-51-like kinase) and mAtg13 (mammalian autophagy-related 13) are phosphorylated, thus inhibiting initiation of autophagy signaling to thus suppress autophagy.

On the other hand, the RAS/Raf/MAPK signal cascade is a signal cascade that is related to cell proliferation, etc. When a ligand such as for example a growth factor binds to a G protein-coupled receptor or a tyrosine kinase receptor, KRAS, which is a low molecular weight G protein, is activated, and the activated KRAS phosphorylates Raf (a kind of MAPKKK) to thus activate it. The activated Raf activates MEK (MAPK/ERK kinase, a kind of MAP2K), and the activated MEK activates ERK (Extracellular signal-regulated kinase, a kind of MAPK). The activated ERK translocates into the nucleus and promotes transcription of various mRNAs to thus trigger cell proliferation.

In the present description, promoting a signal cascade means not only enhancing activation of the signal cascade but also suppression of inactivation of the signal cascade. Whether or not a signal cascade is promoted may be determined by the signal cascade being activated compared with a case in which the agent or composition of the present description is not utilized. Activation of a signal cascade may be evaluated by detecting, for example, but not limited to, a cellular phenomenon resulting from activation of a signal cascade constituent molecule (e.g., phosphorylation, etc.) or activation of a signal cascade, such as for example suppression of autophagy, etc., in the case of the PI3K/Akt/mTOR signal cascade or cell proliferation, etc., in the case of the RAS/Raf/MAPK signal cascade.

The present description also relates to a process for producing an agent or composition for promoting the PI3K/Akt/mTOR signal cascade and/or the RAS/Raf/MAPK signal cascade, the process comprising a step of formulating GST-π and/or a functional variant thereof; use of GST-π and/or a functional variant thereof in the production of an agent or composition for promoting the PI3K/Akt/mTOR signal cascade and/or the RAS/Raf/MAPK signal cascade; GST-π and/or a functional variant thereof for use in the promotion of the PI3K/Akt/mTOR signal cascade and/or the RAS/Raf/MAPK signal cascade; and a method for promoting the PI3K/Akt/mTOR signal cascade and/or the RAS/Raf/MAPK signal cascade, the method comprising administering an effective amount of GST-π and/or a functional variant thereof.

The agent or composition for promoting a signal cascade of the present description is useful for the treatment of a disease associated with an abnormality of the PI3K/Akt/mTOR signal cascade and/or the RAS/Raf/MAPK signal cascade, in particular with a suppression of these signal cascades. Examples of such a disease include, but are not limited to, a disease accompanied by suppression of expression or activity and/or increase in degradation or inactivation of a constituent molecule of these signal cascades (e.g., due to a genetic abnormality of these molecules, suppression of expression or activity of GST-π, etc.).

Therefore, the present description also relates to a pharmaceutical composition for treating a disease associated with suppression of the PI3K/Akt/mTOR signal cascade and/or the RAS/Raf/MAPK signal cascade, the pharmaceutical composition containing as an active ingredient GST-π and/or a functional variant thereof; a process for producing a pharmaceutical composition for treating a disease associated with suppression of the PI3K/Akt/mTOR signal cascade and/or the RAS/Raf/MAPK signal cascade, the process including a step of formulating GST-π and/or a functional variant thereof; use of GST-π and/or a functional variant thereof in the production of a pharmaceutical composition for treating a disease associated with suppression of the PI3K/Akt/mTOR signal cascade and/or the RAS/Raf/MAPK signal cascade; GST-π and/or a functional variant thereof for use in the treatment of a disease associated with suppression of the PI3K/Akt/mTOR signal cascade and/or the RAS/Raf/MAPK signal cascade; and a method for treating a disease associated with suppression of the PI3K/Akt/mTOR signal cascade and/or the RAS/Raf/MAPK signal cascade, the method comprising administering an effective amount of GST-π and/or a functional variant thereof.

The present description also relates to an agent or composition for suppressing the PI3K/Akt/mTOR signal cascade and/or the RAS/Raf/MAPK signal cascade (also called a 'signal cascade suppressing agent' or a 'signal cascade suppressing composition'), the agent or composition containing as an active ingredient a drug that suppresses GST-π.

In the present description, suppressing a signal cascade means not only inducing inactivation of the signal cascade but also suppressing activation of the signal cascade. Whether or not the signal cascade is suppressed may be determined by the signal cascade being suppressed compared with a case in which the agent or composition of the present description is not utilized. Suppression of a signal cascade may be evaluated by detecting, for example, but not limited to, a reduction of activation (e.g., phosphorylation, etc.) of a signal cascade constituent molecule or a cellular phenomenon resulting from suppression of the signal cascade, such as for example increase of autophagy, etc., in the case of the PI3K/Akt/mTOR signal cascade or cell proliferation suppression, etc., in the case of the RAS/Raf/MAPK signal cascade.

The present description also relates to a process for producing an agent or composition for suppressing the PI3K/Akt/mTOR signal cascade and/or the RAS/Raf/MAPK signal cascade, the process comprising a step of formulating a drug that suppresses GST-π; use of a drug that suppresses GST-π in the production of an agent or composition for suppressing the PI3K/Akt/mTOR signal cascade and/or the RAS/Raf/MAPK signal cascade; a drug that suppresses GST-π for use in suppression of the PI3K/Akt/mTOR signal cascade and/or the RAS/Raf/MAPK signal cascade; and a method for suppressing the PI3K/Akt/mTOR signal cascade and/or the RAS/Raf/MAPK signal cascade, the method comprising administering an effective amount of a drug that suppresses GST-π.

The agent or composition for suppressing a signal cascade of the present description is useful for the treatment of a disease associated with an abnormality of the PI3K/Akt/mTOR signal cascade and/or the RAS/Raf/MAPK signal cascade, in particular with activation of these signal cascades. Examples of such a disease include, but are not limited to, a disease associated with an increase in expression or activity and/or suppression of degradation or inactivation of a constituent molecule of these signal cascades (e.g., due to a genetic abnormality of these molecules, an increase in expression or activity of GST-π) and a disease associated with activation of the signal cascade by means of a factor other than a constituent molecule of these signal cascades (e.g., activation of receptor tyrosine kinase, etc.).

Therefore, the present description further relates to a pharmaceutical composition for treating a disease associated with activation of the PI3K/Akt/mTOR signal cascade and/or the RAS/Raf/MAPK signal cascade, the pharmaceutical composition containing as an active ingredient a drug that suppresses GST-π; a process for producing a pharmaceutical composition for treating a disease associated with activation of the PI3K/Akt/mTOR signal cascade and/or the RAS/Raf/MAPK signal cascade, the process comprising a step of formulating a drug that suppresses GST-π; use of a drug that suppresses GST-π in the production of a pharmaceutical composition for treating a disease associated with activation of the PI3K/Akt/mTOR signal cascade and/or the RAS/Raf/MAPK signal cascade; a drug that suppresses GST-π for use in the treatment of a disease associated with activation of the PI3K/Akt/mTOR signal cascade and/or the RAS/Raf/MAPK signal cascade; and a method for treating a disease associated with activation of the PI3K/Akt/mTOR signal cascade and/or the RAS/Raf/MAPK signal cascade, the method comprising administering an effective amount of a drug that suppresses GST-π.

The present description also relates to an agent or composition for suppressing ubiquitination (also called a 'ubiquitination suppressing agent' or a 'ubiquitination suppressing composition'), the agent or composition containing as an active ingredient GST-π and/or a functional variant thereof.

Ubiquitination means that ubiquitin binds to a protein and is involved in the process of disposing of a protein that becomes unnecessary in a cell. A ubiquitinated protein is degraded in a proteasome.

In one embodiment of the present description, the protein for which ubiquitination is suppressed is a protein to which GST-π can bind. Furthermore, in one embodiment of the present description, the protein for which ubiquitination is suppressed is selected from the group consisting of a protein constituting the RAS/Raf/MAPK signal cascade, a protein constituting the PI3K/Akt/mTOR signal cascade, and a tyrosine kinase receptor. In a preferred embodiment of the present description, the protein for which ubiquitination is suppressed is selected from the group consisting of EGFR and Raf-1, in particular a phosphorylated form thereof.

In the present description, suppression of ubiquitination may be determined by ubiquitination being suppressed compared with a case in which the agent or composition of the present description is not utilized. Suppression of ubiquitination may be evaluated by any known technique, for example, but not limited to, an immunoprecipitation method, a western blot method, a mass analysis method, a pull-down method, etc.

The present description also relates to a process for producing an agent or composition for suppressing ubiquitination, the process comprising a step of formulating GST-π and/or a functional variant thereof; use of GST-π and/or a functional variant thereof in the production of an agent or composition for suppressing ubiquitination; GST-π and/or a functional variant thereof for use in the suppression of ubiquitination; and a method for suppressing ubiquitination, the method comprising administering an effective amount of GST-π and/or a functional variant thereof.

The agent or composition for suppressing ubiquitination of the present description is useful in the treatment of a disease associated with hyperubiquitination. Examples of such a disease include, but are not limited to, a disease accompanied by an increase in expression or activity and/or a suppression of degradation or inactivation of ubiquitin ligase (e.g., due to a genetic abnormality of ubiquitin ligase, suppression of expression or activity of GST-π, etc.).

Therefore, the present description further relates to a pharmaceutical composition for treating a disease associated with hyperubiquitination, the pharmaceutical composition containing as an active ingredient GST-π and/or a functional variant thereof; a process for producing a pharmaceutical composition for treating a disease associated with hyperubiquitination, the process comprising a step of formulating GST-π and/or a functional variant thereof; use of GST-π and/or a functional variant thereof in the production of a pharmaceutical composition for treating a disease associated with hyperubiquitination; GST-π and/or a functional variant thereof for use in the treatment of a disease associated with hyperubiquitination; and a method for treating a disease associated with hyperubiquitination, the method comprising administering an effective amount of GST-π and/or a functional variant thereof.

The present description also relates to an agent or composition for promoting ubiquitination (also called a 'ubiquitination promoting agent' or a 'ubiquitination promoting composition'), the agent or composition containing as an active ingredient a drug that suppresses GST-π.

In one embodiment of the present description, the protein for which ubiquitination is promoted is a protein to which GST-π can bind. Furthermore, in one embodiment of the present description, the protein for which ubiquitination is promoted is selected from the group consisting of a protein constituting the RAS/Raf/MAPK signal cascade, a protein constituting the PI3K/Akt/mTOR signal cascade, and a tyrosine kinase receptor. In a preferred embodiment of the present description, the protein for which ubiquitination is promoted is selected from the group consisting of EGFR and Raf-1, in particular a phosphorylated form thereof.

In the present description, promotion of ubiquitination may be determined by ubiquitination being promoted compared with a case in which the agent or composition of the present description is not utilized. Promotion of ubiquitination may be evaluated by any known technique, for example, but not limited to, an immunoprecipitation method, a western blot method, a mass analysis method, a pull-down method, etc.

The present description also relates to a process for producing an agent or composition for promoting ubiquitination, the process comprising a step of formulating a drug that suppresses GST-π; use of a drug that suppresses GST-π in the production of an agent or composition for promoting ubiquitination; a drug that suppresses GST-π for use in the promotion of ubiquitination; and a method for promoting ubiquitination, the method comprising administering an effective amount of a drug that suppresses GST-π.

The agent or composition for promoting ubiquitination of the present description is useful in the treatment of a disease associated with suppression of ubiquitination. Examples of such a disease include, but are not limited to, a disease associated with suppression of expression or activity and/or an increase in degradation or inactivation of ubiquitin ligase (e.g., due to a genetic abnormality of ubiquitin ligase, an increase in expression or activity of GST-π, etc.).

Therefore, the present description further relates to a pharmaceutical composition for treating a disease associated with suppression of ubiquitination, the pharmaceutical composition containing as an active ingredient a drug that suppresses GST-π; a process for producing a pharmaceutical composition for treating a disease associated with suppression of ubiquitination, the process comprising a step of formulating a drug that suppresses GST-π; use of a drug that suppresses GST-π in the production of a pharmaceutical composition for treating a disease associated with suppression of ubiquitination; a drug that suppresses GST-π for use in the treatment of a disease associated with suppression of ubiquitination; and a method for treating a disease associated with suppression of ubiquitination, the method comprising administering an effective amount of a drug that suppresses GST-π.

The present description also relates to an agent or composition for suppressing autophagy (also called an 'autophagy suppressing agent' or an 'autophagy suppressing composition'), the agent or composition containing as an active ingredient GST-π and/or a functional variant thereof. The present inventors have now clarified that GST-π binds to a tyrosine kinase receptor, in particular a phosphorylated form thereof, which is on the upstream of the PI3K/Akt/mTOR signal cascade, to thus inhibit the ubiquitination thereof, thereby promoting the signal cascade; it is known that activation of the PI3K/Akt/mTOR signal cascade suppresses autophagy (e.g., Yang and Klionsky, 2010 supra).

In the present description, suppression of autophagy may be determined by autophagy being suppressed in cells compared with a case in which the agent or composition of the present description is not utilized. The technique for evaluating autophagy is as described above.

The present description further relates to a process for producing an agent or composition for suppressing autophagy, the process comprising a step of formulating GST-π and/or a functional variant thereof; use of GST-π and/or a functional variant thereof in the production of an agent or composition for suppressing autophagy; GST-π and/or a functional variant thereof for use in suppression of autophagy; and a method for suppressing autophagy, the method comprising administering an effective amount of GST-π and/or a functional variant thereof.

The agent or composition for suppressing autophagy of the present description is useful for the treatment of a disease associated with enhanced autophagy. Examples of such a disease include, but are not limited to, a disease associated with suppression of the PI3K/Akt/mTOR signal cascade (e.g., due to a genetic abnormality of a PI3K/Akt/mTOR signal cascade constituent molecule and/or a molecule on the upstream thereof, suppression of expression or activity of GST-π, etc.), myopathy, hepatic damage, reperfusion damage, etc.

Therefore, the present description also relates to a pharmaceutical composition for treating a disease associated with enhanced autophagy, the pharmaceutical composition containing as an active ingredient GST-π and/or a functional variant thereof; a process for producing a pharmaceutical composition for treating a disease associated with enhanced autophagy, the process comprising a step of formulating GST-π and/or a functional variant thereof; use of GST-π and/or a functional variant thereof in the production of a pharmaceutical composition for treating a disease associated with enhanced autophagy; GST-π and/or a functional variant thereof for use in the treatment of a disease associated with enhanced autophagy; and a method for treating a disease associated with enhanced autophagy, the method comprising administering an effective amount of GST-π and/or a functional variant thereof to a subject that requires same.

Furthermore, the present inventors have now clarified that autophagy is promoted by suppressing GST-π. Therefore, the present description also relates to an agent or composition for promoting autophagy (also called an 'autophagy promoting agent' or an 'autophagy promoting composition') containing as an active ingredient a drug that suppresses GST-π. The present description further relates to a process for producing an agent or composition for promoting autophagy, the process comprising a step of formulating a drug that suppresses GST-π; use of a drug that suppresses GST-π in the production of an agent or composition for promoting autophagy; a drug that suppresses GST-π for use in the promotion of autophagy; and a method for promoting autophagy, the method comprising administering an effective amount of a drug that suppresses GST-π.

The agent or composition for promoting autophagy of the present description is useful in the treatment of a disease associated with suppression of autophagy, etc. Examples of such a disease include, but are not limited to, a disease associated with activation of the PI3K/Akt/mTOR signal cascade (e.g., due to a genetic abnormality of a PI3K/Akt/mTOR signal cascade constituent molecule and/or a molecule on the upstream thereof, an increase in expression or activity of GST-π, etc.), aging, and an ischemic disease.

Therefore, the present description also relates to a pharmaceutical composition for treating a disease associated with suppression of autophagy, the pharmaceutical composition containing as an active ingredient a drug that suppresses GST-π; a process for producing a pharmaceutical composition for treating a disease associated with suppression of autophagy, the process comprising a step of formulating a drug that suppresses GST-π; use of a drug that suppresses GST-π in the production of a pharmaceutical composition for treating a disease associated with suppression of autophagy; a drug that suppresses GST-π for use in the treatment of a disease associated with suppression of autophagy; and a method for treating a disease associated with suppression of autophagy, the method comprising administering an effective amount of a drug that suppresses GST-π to a subject that requires same.

The formulation amount of the active ingredient of the various types of agent or composition of the present description related to the suppression/promotion of a signal cascade, ubiquitination, or autophagy may be an amount that achieves a desired effect (i.e., suppression/promotion of the signal cascade, ubiquitination, or autophagy) when the agent or composition is administered. Furthermore, it is preferably an amount that does not cause an adverse effect that exceeds the benefit of administration. Such an amount is known or may be determined appropriately by means of an in vitro test using cultured cells, etc., or a test in a model animal such as a mouse, a rat, a dog, or a pig, and such a test method is well known to a person skilled in the art. Inhibition/promotion of a signal cascade, ubiquitination, or autophagy may be evaluated by various known techniques, including those described above. The formulation amount of active ingredient can vary according to the mode of administration of the agent or composition. For example, when a plurality of units of the composition is used for one administration, the amount of active ingredient to be formulated in one unit of the composition may be one obtained by dividing the amount of active ingredient necessary for one administration by said plurality of units. Adjustment of such a formulation amount can be carried out appropriately by a person skilled in the art.

The drug and the formulation amount thereof in the production process or use of the various types of agent or composition related to suppression/promotion of a signal cascade, ubiquitination, or autophagy are as described above. Formulation of each drug may be carried out in accordance with any known technique.

All of the various types of methods related to suppression/promotion of a signal cascade, ubiquitination, or autophagy may be an in vitro method or an in vivo method. Furthermore, the effective amount of drug in the above methods may be an amount that achieves a desired effect (i.e., suppression/promotion of a signal cascade, ubiquitination, or autophagy) in cells to which it is administered. Moreover, it is preferably an amount that does not cause an adverse effect that exceeds the benefit of administration. Such an amount is known or may be determined appropriately by an in vitro test, etc., using cultured cells, etc., and such a test method is well known to a person skilled in the art. Achievement of a desired effect may be evaluated by various known techniques, including those described above. The effective amount above need not necessarily be one that induces a desired effect in all the cells of a cell population to which the drug is administered. For example, the effective amount above may be an amount that induces a desired effect in, of the cell population, at least 1% of cells, at least 2%, at least 3%, at least 4%, at least 5%, at least 6%, at least 8%, at least 10%, at least 12%, at least 15%, at least 20%, at least 25%, etc.

When the active ingredient in the various agents or compositions, treatment methods, etc., of the present invention described herein is a nucleic acid, for example, an RNAi molecule, a ribozyme, an antisense nucleic acid, a DNA/RNA chimera polynucleotide, etc., it may be used as a naked nucleic acid as it is, but may also be carried by various vectors. As the vector, any known vector such as a plasmid vector, a phage vector, a phagemid vector, a cosmid vector, or a virus vector may be used. The vector preferably contains at least a promoter that enhances expression of the nucleic acid carried, and in this case the nucleic acid is preferably operably linked to such a promoter. The nucleic acid being operably linked to a promoter referred to herein means that the nucleic acid and the promoter are positioned so that a protein encoded by the nucleic acid is appropriately produced by the action of the promoter. The vector may or may not be replicable in a host cell, and the transcription of a gene may be carried out either outside the nucleus or within the nucleus of a host cell. In the latter case, the nucleic acid may be incorporated into the genome of a host cell.

Furthermore, the active ingredient may be carried by various non-viral lipid or protein carriers. Examples of such carriers include, but are not limited to, cholesterol, a liposome, an antibody protomer, cyclodextrin nanoparticles, a fusion peptide, an aptamer, a biodegradable polylactic acid copolymer, and polymer; the efficiency of incorporation into cells can be enhanced (see, e.g., Pirollo and Chang, Cancer Res. 2008; 68 (5): 1247-50, etc.). In particular, a cationic liposome or a polymer (e.g., polyethyleneimine, etc.) is useful. Further examples of useful polymers as such a carrier include those described in US 2008/0207553, US 2008/0312174, etc.

With regard to the various pharmaceutical compositions of the present invention described herein, the active ingredient may be combined with another optional component as long as the effect of the active ingredient is not impaired. Examples of such an optional component include another chemical therapeutic agent, a pharmacologically acceptable carrier, an excipient, a diluent, etc. Furthermore, depending on the route of administration, the mode of drug release, etc., the composition may be coated with an appropriate material such as for example an enteric coating or a timed disintegration material, or may be incorporated into an appropriate drug release system.

The various agents and compositions (including the various pharmaceutical compositions) of the present invention described herein may be administered via various routes including both oral and parenteral routes, for example, without limitation, oral, intravenous, intramuscular, subcutaneous, local, intratumoral, rectal, intraarterial, intraportal, intraventricular, transmucosal, transdermal, intranasal, intraperitoneal, intrapulmonary, and intrauterine routes, and may be formulated into a dosage form suitable for each administration route. With regard to such dosage forms and formulation methods, any known form or method may be employed appropriately (see, e.g., Hyojun yakuzaigaku (Standard Pharmaceutical Science), Ed. by Yoshiteru Watanabe et al., Nankodo, 2003, etc.).

Examples of the dosage form suitable for oral administration include, but are not limited to, a powder, granules, a tablet, a capsule, a liquid, a suspension, an emulsion, a gel, and a syrup, and examples of the dosage form suitable for parenteral administration include an injection such as a solution injection, a suspension injection, an emulsion injection, or an injection in a form that is prepared at the time of use. A formulation for parenteral administration may be in the form of an aqueous or nonaqueous isotonic sterile solution or suspension.

The various agents or compositions (including various pharmaceutical compositions) of the present description described herein may be targeted at a specific tissue or cells. Targeting may be achieved by any known technique. When delivery to a cancer is attempted, for example, without limitation, a technique such as passive targeting in which a formulation is made into a size of 50 to 200 *µ*m in diameter, in particular 75 to 150 *µ*m, etc., which is suitable for exhibition of an EPR (enhanced permeability and retention) effect, or active targeting in which a ligand of CD19, HER2, a transferrin receptor, a folic acid receptor, a VIP receptor, EGFR (Torchilin, AAPS J. 2007; 9 (2) : E128-47), RAAG10 (JP, A (PCT) 2005-532050), PIPA (JP, A (PCT) 2006-506071), or KID3 (JP, A (PCT) 2007-529197), etc., a peptide having an RGD motif or an NGR motif, F3, LyP-1 (Ruoslahti et al., J Cell Biol. 2010; 188 (6): 759-68), etc. , is used as a targeting agent may be used. Furthermore, since a retinoid is known to be useful as a targeting agent for cancer cells (WO 2008/120815), a carrier containing a retinoid as a targeting agent may also be used. Such carriers are described in the literature above as well as in WO 2009/036368, WO 2010/014117, etc.

The various agents or compositions (including various pharmaceutical compositions) of the present invention described herein may be supplied in any form, and from the viewpoint of storage stability, may be provided in a form that can be prepared at the time of use, for example, a form that allows a doctor and/or pharmacist, a nurse, another paramedic, etc. , to prepare it at the medical site or its vicinity. Such a form is particularly useful when the agent or composition of the present invention contains a component that is difficult to store stably, such as a lipid, a protein, or a nucleic acid. In this case, the agent or composition of the present invention is provided in one or more containers containing at least one of the essential constituents, and preparation is carried out prior to use, for example, within 24 hours, preferably within 3 hours, and more preferably immediately before use. When carrying out preparation, a reagent, a solvent, preparation equipment, etc. , that are usually available at a place of preparation may be used as appropriate.

Therefore, the present description also relates to a kit for preparing a composition, the kit containing one or more containers, the container singly or in combination containing active ingredients to be contained in the various agents or compositions of the present description and essential constituents of the various agents or compositions provided in the form of such a kit. The kit of the present description may include, in addition to the above, instructions such as a written explanation or an electronic recording medium such as a CD or DVD describing a preparation method, an administration method, etc., for the various agents or compositions of the present description. Furthermore, the kit of the present description may contain all of the constituents for completing the various agents or compositions of the present description, but need not necessarily contain all of the constituents. Therefore, the kit of the present description need not contain a reagent or a solvent that is usually available at a medical site, an experimental laboratory, etc., such as sterile water, physiological saline, or a glucose solution.

The effective amount of the various treatment methods of the present description described herein is for example an amount that reduces symptoms of a disease or delays or stops the progress of a disease, and is preferably an amount that suppresses or cures a disease. It is also preferably an amount that does not cause an adverse effect that exceeds the benefit of administration. Such an amount may be determined appropriately by an in vitro test using cultured cells, etc. , or a test in a model animal such as a mouse, a rat, a dog, or a pig, and such test methods are well known to a person skilled in the art. Furthermore, the dose of a drug used in the treatment method of the present description is known to a person skilled in the art or may be determined appropriately by the tests described above, etc.

The specific dose of the active ingredient to be administered in the treatment method of the present description described herein can be determined by taking into consideration various conditions related to the subject that requires treatment, such as for example the seriousness of symptoms, the general health state of the subject, age, body weight, the gender of the subject, diet, the timing and frequency of administration, concomitant pharmaceuticals, the responsiveness to the treatment, the dosage form, and compliance with the treatment.

Examples of the administration route include various routes, including both oral and parenteral routes, such as oral, intravenous, intramuscular, subcutaneous, local, intratumoral, rectal, intraarterial, intraportal, intraventricular, transmucosal, transdermal, intranasal, intraperitoneal, intrapulmonary, and intrauterine routes.

The frequency of administration depends on the properties of the agent or composition used and the condition of the subject, including those described above, and may be a plurality of times a day (that is, two, three, four, five, or more times a day), once a day, every few days (that is, every two, three, four, five, six, seven days, etc.), every week, every few weeks (that is, every two, three, four weeks, etc.), etc.

When used herein, the term 'subject' means any biological individual and is preferably an animal, more preferably a mammal, and yet more preferably a human individual. In the present invention, the subject may be either healthy or affected by some disease, but when an attempt is made to treat a specific disease, it typically means a subject affected with such a disease or having a risk of being affected.

Furthermore, when used herein, the term 'treatment' includes all types of preventive and/or therapeutic interventions medically allowed for the purpose of cure, temporary remission, prevention, etc., of a disease. For example, the term 'treatment' includes medically allowable interventions for various types of purposes including delaying or stopping the progress of a disease, making a lesion regress or disappear, preventing onset, or inhibiting recurrence.

### [Examples]

The present invention is further explained below based on Examples, but such Examples are only illustrations of the present invention and should not be construed as limiting the present invention.

### Example 1: Effect of GST-π knockout on RAS/Raf/MAPK signal cascade

### (1) Cell culturing

K-RAS mutation-positive colon carcinoma cell line M7609 was cultured in 10% fetal bovine serum (FBS)-containing RPMI-1640 medium at 37°C under an atmosphere containing 5% CO₂. Furthermore, 100 U/mL penicillin and 100 *µ*g/mL streptomycin were added as antibiotics to the medium.

### (2) Transfection of GST-π siRNA

On the day before transfection, M7609 cells were plated on a 100 mm plastic tissue culture dish using 10% FBS-containing RPMI-1640 medium containing no antibiotic so as to give 1 × 10⁶ cells/10 mL. 600 pmol of GST-π siRNA (SEQ ID No: 1: GGGAGGCAAGACCUUCAUUTT, siRNA ID#2385, Ambion) was added to 1 mL of Opti-MEM I Reduced Serum Medium (GIBCO) and mixed gently. Subsequently, 35 *µ*L of Lipofectamine RNAiMAX (Invitrogen) was diluted in 1 mL of Opti-MEM I Reduced Serum Medium and mixed gently. The diluted GST-π siRNA and the diluted Lipofectamine RNAiMAX were combined and gently mixed, and then incubated at room temperature for 10 min. During this time, the medium was replaced with 10 mL of Opti-MEM I Reduced Serum Medium. After the 10 min. incubation, the complex between GST-π siRNA and Lipofectamine RNAiMAX was added to the cells and incubated at 37°C under an atmosphere containing 5% CO₂. After 5 hours incubation, it was replaced with 10 mL of 10% FBS-containing RPMI-1640 medium containing no antibiotic. As a control experiment, the same procedure was repeated using Scramble siRNA (SEQ ID No: 2: CGAUUCGCUAGACCGGCUUCAUUGCAG, Hokkaido System Science Co., Ltd.). On the 1st, 2nd, 3rd, and 4th days after transfection with GST-π siRNA, the number of cells was counted, and knockdown of GST-π was examined. Knockdown of GST-π was analyzed by a western blot method as described below.

### (3) Western blot analysis of GST-π knockdown

Western blot analysis of GST-π knockdown was carried out using cells harvested at above-mentioned each point of time after transfection with GST-n siRNA. The harvested cells were cultured for 16 hours in a serum-free medium. After the cells were washed with cold PBS, cold lysis buffer (1% NP-40, 50 mM Tris-HCl, 150 mM NaCl, 1 mM EDTA, complete Mini EDTA-free (Roche), PhosSTOP (Roche), pH 7.5) was added thereto, and solubilization was carried out by incubating for 30 min. while cooling with ice. Centrifuging was carried out at 4°C and 15000 rpm for 15 min., thus giving a cell extract. The cell extract thus obtained was subjected to quantitative protein analysis using a Micro BCA Protein Assay Kit (Thermo SCIENTIFIC) (GST-π siRNA transfectant: 4.35 *µ*g/*µ*L, Scramble siRNA transfectant: 4.56 *µ*g/*µ*L). Subsequently, 20 *µ*g of the cell extract was denatured under reducing conditions, and the protein was separated by carrying out SDS-PAGE using multi gel II Mini 4/20 (13W) (Cosmo Bio Co., Ltd.). After SDS-PAGE was completed, transfer to a PVDF membrane was carried out electrically using a tank-type blotting system. The transfer membrane was blocked by incubating with 5% skimmed milk/0.05% Tween20 in PBS (abbreviated to PBS-T) at 4°C for 16 hours. Subsequently, a reaction with PBS-T-diluted anti-GST-n antibody (MBL) was carried out at 4°C for 16 hours. A secondary antibody reaction was carried out using horseradish peroxidase (HRP)-labeled rabbit antibody at room temperature for 1 hour. A reaction with a chemiluminescent substrate was then carried out at room temperature for 1 min., and then this chemiluminescence was detected using an X-ray film. Washing between operations was carried out three times by shaking for 5 min using PBS-T. Furthermore, after transfection with siRNA, plating on 60 mm plastic tissue culture dish was carried out to give 1.0 × 10⁵ cells/5 mL, and the total cell count in the dish was measured using a hemocytometer up to the 4th day.

### (4) Western blot analysis of protein involved in RAS/Raf/MAPK signal cascade

A western blot analysis for the main protein involved in the RAS/Raf/MAPK signal cascade was carried out in the same way as for (3) above using cells harvested on the 2nd day after transfection with GST-π siRNA. As antibodies, in addition to anti-GST-n antibody, anti-p-Raf-1 (Ser338) antibody (MILLIPORE), anti-Raf-1 antibody (Santa Cruz), anti-p-MEK1/2 (Ser217/221) antibody (Cell Signaling), anti-MEK1/2 antibody (Cell Signaling), anti-p-ERK1/2 (Thr202/Tyr204) antibody (Cell Signaling), anti-ERK antibody (Cell Signaling), and anti-GAPDH antibody (Abcam) were used.

The results are shown in FIGS. 1 and 2. In FIG. 1 a), it was observed that expression of GST-π was suppressed by GST-π siRNA, but it was not suppressed by Scramble siRNA. In FIG. 1 b), it was found that even at the point of time when 4 days had elapsed after transfection, expression of GST-π was still stably suppressed by GST-π siRNA and, furthermore, in the case of expression of GST-π being suppressed, the number of cells after culturing for 4 days was markedly less than in the case of no suppression. Furthermore, it was also evident from FIG. 2 that in the GST-π siRNA treated group, phosphorylation of all proteins involved in the RAS/Raf/MAPK signal cascade decreased compared with the Scramble siRNA treated group. It is therefore clear that, due to suppression of expression of GST-π, the RAS/Raf/MAPK signal cascade and cell proliferation abnormality were suppressed.

### Example 2: Effect of GST-π knockout on ubiquitination

### (1) Effect of GST-π knockdown on ubiquitination of Raf-1

Culturing of M7609 cells and transfection with GST-π siRNA were carried out in accordance with the procedures of Example 1 (1) and (2).

In the same way as for Example 1 (3), on the 2nd day after siRNA transfection culturing was carried out for 16 hours in a serum-free medium, and a cell extract was collected. The cell extract thus obtained was subjected to quantitative protein analysis using a Micro BCA Protein Assay Kit (Scramble siRNA: 8.88 *µ*g/*µ*L, GST-π siRNA: 7.18 *µ*g/ mL). 0.5 *µ*g of the cell extract was mixed with anti-Raf-1 antibody conjugated to Dynabeads Protein G (Invitrogen), incubation was carried out at 4°C for 2 hours while gently mixing in a shaker to thus isolate Raf-1 protein, and then a western blot analysis was carried out in the same way as for Example 1 (3) using an anti-ubiquitin antibody (Santa Cruz). Phosphorylation modification of Ser621, which is involved in inhibition of Raf-1 proteasome degradation, was investigated in the same manner using an anti-p-Raf-1 (Ser621) antibody (MILLIPORE).

### (2) Effect of proteasome inhibitor on Raf-1 expression under GST-π knockdown

Culturing of M7609 cells and transfection with GST-π siRNA were carried out in accordance with the procedures of Example 1 (1) and (2). On the 2nd day after GST-π siRNA transfection culturing was carried out for 16 hours in a serum-free medium. After treating with 5 *µ*M MG132 for 4 hours, a cell extract was collected. As a control for the MG132 treatment, treatment with 0.05% DMSO was carried out in the same manner. The cell extract thus obtained was subjected to quantitative protein analysis using a Micro BCA Protein Assay Kit (Scramble siRNA-DMSO treated group: 3.36 *µ*g/*µ*L, Scramble siRNA-MG132 treated group: 3.16 *µ*g/*µ*L, GST-π siRNA-DMSO treated group: 3.12 *µ*g/*µ*L, GST-π siRNA-MG132 treated group: 3.16 *µ*g/*µ*L). 20 *µ*g of the cell extract was subjected to SDS-PAGE, and then western blot analysis was carried out in the same way as for Example 1 (3) using an anti-p-Raf-1 (Ser338) antibody and an anti-Raf-1 antibody.

### (3) Co-immunoprecipitation of p-Raf-1 and GST-π

Culturing of M7609 cells was carried out in accordance with the procedure of Example 1 (1). Subsequently, after the GST-π knockdown cells obtained by the procedure of Example 1 (2) were washed with cold PBS, a cold co-immunoprecipitation buffer (0.5% NP-40, 50 mM HEPES, 150 mM NaCl, 1 mM EGTA, 1.5 mM MgCl₂, complete Mini EDTA-free, PhosSTOP, pH 7.5) was added, and solubilization was carried out by incubating for 30 min. while ice cooling. Centrifuging was carried out at 4°C and 15000 rpm for 15 min., thus giving a cell extract. The cell extract thus obtained was subjected to quantitative protein analysis using a Micro BCA Protein Assay Kit (12.1 *µ*g/*µ*L). 1 mg of the cell extract was mixed with anti-p-Raf-1 (Ser338) antibody (US Biological) conjugated to Dynabeads Protein G, and incubation was carried out at 4°C for 16 hours while mixing gently in a shaker, thus carrying out co-immunoprecipitation. Subsequently, a western blot analysis was carried out using anti-GST-π antibody.

The results are shown in FIGS. 3 to 5. It was clear from FIG. 3 that in the GST-π siRNA treated group the amount of ubiquitin co-precipitated with Raf-1 was large compared with the Scramble siRNA treated group, and ubiquitination of Raf-1 was enhanced. Furthermore, it can be seen from FIG. 4 that proteasome was involved in reduction of the expression of p-Raf-1 by GST-π siRNA, and it can be seen from FIG. 5 that GST-π was bound to p-Raf-1. The above results suggest that GST-π binds to p-Raf-1 to thus inhibit its ubiquitination, and due to suppression of GST-π, ubiquitination of p-Raf-1 is promoted, and the abundance of p-Raf-1 is reduced.

### Example 3: Analysis of induction of autophagy and apoptosis by GST-π knockdown

### (1) Analysis of induction of autophagy by immunofluorescence staining

Induction of autophagy by GST-π knockdown was analyzed by immunofluorescence staining with LC3, which is an autophagy-specific marker protein. GST-π knockdown cells obtained by the procedure of Example 1 (2) were plated on a cover slip placed in a 35 mm plastic tissue culture dish so as to give 1 × 10⁵ cells/2 mL. After the medium was aspirated, 4% paraformaldehyde in PBS was added, and incubation was carried out at room temperature for 10 min., thus fixing the cells. Permeabilization of the cells was carried out using 0.5% Triton X-100 in PBS on ice for 5 min. After washing with cold PBS for 10 min., a reaction with anti-LC3B antibody (Invitrogen) diluted with 1% BSA containing PBS was carried out within a humidity chamber at 37°C for 1 hour. A secondary antibody reaction was carried out using Alexa Fluor488-labeled rabbit antibody (Invitrogen) at 37°C for 1 hour. Mounting on a slide glass was carried out using Prolong Gold antifade reagent with DAPI, and incubation was carried out at 4°C for 16 hours. Washing after the antibody reaction was carried out three times using PBS at 37°C for 5 min. Autophagy-positive cells at the time of examination with a fluorescence microscope were defined as cells having a spot-like LC3 signal present in the cytoplasm.

### (2) Analysis of induction of autophagy by western blotting

Induction of autophagy in GST-π knockdown cells was further analyzed by a western blot analysis of LC3. GST-π knockdown cells obtained by the procedure of Example 1 (2) were plated on a cover slip placed in a 35 mm plastic tissue culture dish so as to give 1 × 10⁵ cells/2 mL. After incubating for a predetermined time, a cell extract was collected and subjected to a western blot analysis. A reaction with a transfer membrane was carried out at 4°C for 16 hours using anti-LC3B antibody (SIGMA) as a primary antibody. Detection of LC3 molecules was carried out using a chemoluminescent reagent after a reaction with HRP-labeled secondary antibody. Whether or not autophagy was induced was evaluated by a shift of LC3 from type I (18 kDa) to type II (16 kDa).

### (3) Analysis of induction of autophagy by examination with electron microscope

On the 1st day after siRNA transfection in Example 1 (2), the cells were plated on an 8-well culture slide for tissue culture so as to give 0.4 × 10⁵ cells/0. 5 mL. Fixation was carried out using 2.5% glutaraldehyde in 0.1 M cacodylic acid buffer (pH 7.4) for 1 hour, and after rinsing with 0.1 M cacodylic acid buffer post-fixation was carried out using 1% OsO₄ and 1.5% potassium ferrocyanide for 2 hours. After dehydration was carried out three times with ethanol for 10 min., embedding in an epoxy resin (TAAB Laboratories Equipment) was carried out. An ultrathin section was prepared using a diamond knife, electron staining was carried out using uranyl acetate and lead citrate, and the section was examined using an electron microscope (Hitachi Transmission Electron Microscope H-7500, Hitachi High-Technologies Corporation).

### (4) Analysis of induction of apoptosis by TUNEL staining

A TUNEL method was carried out as follows using an In Situ Cell Death Detection Kit, POD (Roche). GST-π knockdown cells obtained by the procedure of Example 1 (2) were plated on a cover slip placed in a 35 mm plastic tissue culture dish so as to give 1 × 10⁵ cells/2 mL. After the medium was aspirated, 4% paraformaldehyde in PBS was added, and incubation was carried out at room temperature for 60 min., thus fixing the cells. In order to carry out blocking of endogenous peroxidase, incubation was carried out with 3% H₂O₂ in methanol at room temperature for 10 min. Subsequently, permeabilization of cells was carried out by treating with 0.1% Triton X-100 in 0.1% sodium citrate on ice for 2 min. The TUNEL reaction was carried out in a humidity chamber at 37°C for 60 min. Detection of TUNEL-positive cells was carried out by a coloration reaction using a DAB substrate after reacting a peroxidase labeled anti-fluorescein antibody at 37°C for 30 min. Counter staining was carried out using hematoxylin, stained cells were examined under an optical microscope, and TUNEL-positive cells were evaluated as apoptotic cells. Washing between the operations was carried out by rinsing with PBS.

The results are shown in FIGS. 6 to 10. FIG. 6 is an image of immunofluorescence staining with anti-LC3 antibody, and in the cells shown by arrows a spot-like signal showing LC3 was observed. Since this LC3 spot-like signal would be autophagosome, it can be seen that in the GST-π knockdown cells autophagy was induced.

FIG. 7 shows images of electron microscope examination of the GST-π KD cells 2 days after GST-π siRNA transfection. The right-hand image is an enlargement of part A surrounded by a square in the left-hand image. In the right-hand image, it can be seen that in parts shown by arrows, autophagosome was formed so as to surround the mitochondria.

FIG. 8 shows the results of LC3 western blotting. There are two types of LC3 protein that are recognized by an anti-LC3 antibody. When autophagy occurs and LC3 is incorporated into a lipid double membrane, LC3 changes from type I to type II. Therefore, it is possible to confirm whether or not autophagy has been induced from a change in the amount of LC3-type II detected. From the result of FIG. 8, it can be seen that in the GST-π siRNA treated group, expression of both type I and type II LC3 was induced and type II was markedly increased, whereas in the Scramble siRNA treated group, expression was low for both type I and type II and the level of expression of type I was lower than that of type II.

From these results, it can be seen that autophagy is induced by suppressing expression of GST-π.

FIG. 9 shows the result of TUNEL staining. The upper row shows images of the Scramble siRNA treated group, the lower row shows images of the GST-π siRNA treated group; in the cells in which GST-π was knocked down, TUNEL-positive cells, that is, apoptosis, was observed.

FIG. 10 is a graph showing change over time of the proportions of autophagy-positive cells and apoptosis-positive cells in the GST-π siRNA treated group and the Scramble siRNA treated group. The proportion of autophagy-positive cells denotes the proportion of cells having a spot-like LC3 signal per 500 cells in the immunofluorescence staining experiment using the anti-LC3 antibody in (1) above, and the proportion of apoptosis-positive cells denotes the proportion of TUNEL-positive cells per 1000 cells in the TUNEL staining experiment in (4) above. It can be seen from this that the proportion of autophagy-positive cells increased rapidly after treatment, peaked on 2nd day, and then decreased, whereas the proportion of apoptosis-positive cells gradually but continuously increased up to 4th day.

### Example 4: Effect of GST-π knockout on EGFR/PI3K/Akt/mTOR signal cascade

### (1) Western blot analysis of EGFR/PI3K/Akt/mTOR signal

Expression of each protein constituting the EGFR/PI3K/Akt/mTOR signal cascade was analyzed in the same way as for Example 1 (1), (2), and (4) except that, as primary antibodies, anti-p-EGFR (Tyr1068) antibody (Cell Signaling), anti-EGFR antibody (Santa Cruz), anti-p-PI3K p85 (Tyr458)/p55 (Tyr199) antibody (Cell Signaling), anti-PI3K, p85 antibody (MILLIPORE), anti-p-Akt (Ser473) antibody (Cell Signaling), anti-Akt antibody (Cell Signaling), anti-p-p70S6K (Thr389) antibody (Cell Signaling), anti-p-p70S6K (Thr421/Ser424) antibody (Cell Signaling), and anti-p70S6K antibody (Cell Signaling) were used.

### (2) Effect of proteasome inhibitor on p-EGFR expression under GST-π knockout

Culturing of M7609 cells and transfection with GST-π siRNA were carried out in accordance with the procedures of Example 1 (1) and (2). On the 2nd day after transfection with GST-π siRNA, the cells were cultured in serum-free medium for 16 hours. After treating with 5 *µ*M MG132 for 2 hours, a cell extract was collected. As a control for the MG132 treatment, treatment with 0.05% DMSO was carried out in the same manner. The cell extract thus obtained was subjected to quantitative protein analysis using a Micro BCA Protein Assay Kit (Scramble siRNA-DMSO treated group: 11.98 *µ*g/*µ*L, Scramble siRNA-MG132 treated group: 12.29 *µ*g/*µ*L, GST-π siRNA-DMSO treated group: 8.91 *µ*g/*µ*L, GST-π siRNA-MG132 treated group: 9.24 *µ*g/*µ*L). After 80 *µ*g of the cell extract was subjected to SDS-PAGE, a western blot analysis was carried out using anti-p-EGFR (Tyr1068) antibody and anti-EGFR antibody.

### (3) Co-immunoprecipitation of p-EGFR and GST-π

Culturing of M7609 cells was carried out in accordance with the procedure of Example 1 (1). Subsequently, after the cells were washed with cold PBS, a cold co-immunoprecipitation buffer (1.0% Triton X-100, 50 mM Tris-HCl, 150 mM NaCl, complete Mini EDTA-free, PhosSTOP, pH 7.5) was added, and solubilization was carried out by incubating for 30 min. while ice-cooling. Centrifuging was carried out at 4°C and 12000 rpm for 10 min., thus giving a cell extract. The cell extract thus obtained was subjected to quantitative protein analysis using a Micro BCA Protein Assay Kit (9.08 *µ*g/*µ*L). 1 mg of the cell extract was mixed with anti-p-EGFR (Tyr1068) antibody (Calbiochem) conjugated to Dynabeads Protein G, and co-immunoprecipitation was carried out by incubating in a shaker at 4°C for 16 hours while mixing gently. Subsequently, a western blot analysis was carried out using anti-GST-π antibody.

The results are shown in FIGS. 11 to 13. FIG. 11 shows that in the GST-π siRNA treated group phosphorylation of each protein constituting the EGFR/PI3K/Akt/mTOR signal cascade was reduced compared with the Scramble siRNA treated group, FIG. 12 shows that proteasome is involved in reduction of the expression of p-EGFR by GST-π siRNA, and FIG. 13 shows that GST-π is bound to p-EGFR. The results above suggest that GST-π binds to p-EGFR to thus contribute to stabilization thereof, ubiquitination of p-EGFR is promoted by suppression of GST-π, and the abundance of p-EGFR decreases.

### Example 5: Effect of GST-π inhibitor on cell proliferation, etc.

### (1) Effect of GST-π inhibitor on phosphorylation of EGFR and Raf-1

Culturing of M7609 cells was carried out in accordance with the procedure of Example 1 (1). The cells were plated on a 60 mm plastic tissue culture dish so as to give 4 . 0 × 10⁵ cells/5 mL. GST-π inhibitor C16C2 (prepared by Teijin Pharma Limited per request) was added so as to give 10, 50, and 100 *µ*M, and after 24 hours a cell extract was collected. The cell extract thus obtained was subjected to quantitative protein analysis using a Micro BCA Protein Assay Kit (non-treated: 8.5 *µ*g/*µ*L, 10 *µ*L: 8.49 *µ*g/*µ*L, 50 *µ*M: 7.68 *µ*g/*µ*L, 100 *µ*M: 6.4 *µ*g/*µ*L). 50 *µ*g of the cell extract was subjected to SDS-PAGE, and expression of each protein was then analyzed by the western blotting method.

### (2) Effect of GST-π inhibitor on cell proliferation, etc.

Culturing of M7609 cells was carried out in accordance with the procedure of Example 1 (1). The cells were plated on a 60 mm plastic tissue culture dish so as to give 1.0 × 10⁵ cells/5 mL. After 16 hours, the GST-n inhibitor was added so as to give 50 *µ*M. The total count of cells in the dish was measured using a hemocytometer up to the 2nd day. As a control for the GST-π inhibitor, treatment with 0.05% DMSO was carried out.

The results are shown in FIGS. 14 and 15. It has become clear from these results that the GST-π inhibitor could also suppress phosphorylation of EGFR and Raf-1 (FIG. 14) and cell proliferation (FIG. 15) in the same way as with GST-π knockdown.

### Example 6: Effect of autophagy inhibitor on GST-π knockdown cells

### (1) Cell culturing

Transfection of GST-π siRNA was carried out in accordance with the procedure of Example 1 (2), the medium was then replaced with an antibiotic-free medium, and incubation was carried out for 3 hours. Cells were plated on a cover slip placed in a 35 mm plastic tissue culture dish, the autophagy inhibitor 3-methyl adenine (3-MA, SIGMA) was added so as to give 1 or 5 mM, and following this culturing was carried out for a predetermined time.

### (2) Evaluation of autophagy-positive cells

The cells cultured in (1) were subjected to immunofluorescence staining using anti-LC3 antibody in the same way as for Example 3 (1).

### (3) TUNEL staining

The cells cultured in (1) were subjected to TUNEL staining in the same way as for Example 3 (4).

The results are shown in FIGS. 16 to 18. FIG. 16 shows the proportion of autophagy-positive cells per 1000 cells in each group, and it can be seen that the proportion of autophagy-positive cells was markedly decreased by the autophagy inhibitor. In FIG. 17, the upper row shows the GST-π siRNA + 1 mM 3-MA treated group and the lower row shows the GST-π siRNA + 5 mM 3-MA treated group. It can be seen from FIG. 9 and FIG. 17 that apoptosis-positive cells increased markedly due to the use of an autophagy inhibitor in combination. FIG. 18 is a graph showing that apoptosis was further induced by autophagy inhibitor in a dose-dependent manner. Apoptosis was induced by the addition of GST-π siRNA, but when 3-MA, which is an autophagy inhibitor, was further added, apoptosis was further induced dependent on the additional dose of 3-MA. Therefore, it has become clear that apoptosis can be induced more efficiently by combining a drug that suppresses GST-π and a drug that suppresses autophagy.

## Claims

1. A composition for the use in the treatment of cancer due to GST-π overexpression and KRAS mutation, wherein the composition comprises as active ingredients a drug that suppresses GST-π and a drug that suppresses autophagy, wherein the drug that suppresses autophagy is a PI3K inhibitor, and wherein the PI3K inhibitor is a class III PI3K inhibitor.

2. The composition according to claim 1 for use according to claim 1, wherein the PI3K inhibitor is 3-methyladenine.

3. The composition according to claim 1 for the use according to claim 1, wherein the PI3K inhibitor is selected from the group consisting of an RNAi molecule, a ribozyme, an antisense nucleic acid, a DNA/RNA chimera polynucleotide, and a vector expressing same.

4. The composition according to any one of the claims above for the use according to any one of the claims above, wherein the drug that suppresses GST-π is selected from the group consisting of an RNAi molecule, a ribozyme, an antisense nucleic acid, a DNA/RNA chimera polynucleotide, and a vector expressing same.

## Patentansprüche

1. Zusammensetzung zur Verwendung in der Behandlung von Krebs wegen GST-π Überexprimierung und KRAS-Mutation, wobei die Zusammensetzung als Wirkstoffe einen Arzneistoff, welcher GST-π unterdrückt und einen Arzneistoff, welcher Autophagie unterdrückt, umfasst, wobei der Arzneistoff, welcher Autophagie unterdrückt, ein PI3K-Inhibitor ist, und wobei der PI3K-Inhibitor ein Klasse-III-PI3K-Inhibitor ist.

2. Zusammensetzung gemäß Anspruch 1 zur Verwendung gemäß Anspruch 1, wobei der PI3K-Inhibitor 3-Methyladenin ist.

3. Zusammensetzung gemäß Anspruch 1 zur Verwendung gemäß Anspruch 1, wobei der PI3K-Inhibitor ausgewählt wird aus der Gruppe bestehend aus einem RNAi-Molekül, einem Ribozym, einer Antisense-Nukleinsäure, einem DNA/RNA-Chimären-Polynukleotid, und einem selbige(s) exprimierenden Vektor.

4. Zusammensetzung gemäß irgendeinem der vorstehenden Ansprüche zur Verwendung gemäß irgendeinem der vorstehenden Ansprüche, wobei der Arzneistoff, welcher GST-π unterdrückt, ausgewählt wird aus der Gruppe bestehend aus einem RNAi-Molekül, einem Ribozym, einer Antisense-Nukleinsäure, einem DNA/RNA-Chimären-Polynukleotid, und einem selbige(s) exprimierenden Vektor.

## Revendications

1. Composition pour une utilisation dans le traitement d'un cancer dû à une surexpression de GST-π et à une mutation de KRAS, la composition comprenant en tant que principes actifs un médicament qui supprime la GST-π et un médicament qui supprime une autophagie, dans laquelle le médicament supprimant une autophagie est un inhibiteur de la PI3K, et dans lequel l'inhibiteur de la PI3K est un inhibiteur de la PI3K de classe III.

2. Composition selon la revendication 1 pour une utilisation selon la revendication 1, dans laquelle l'inhibiteur de la PI3K est la 3-méthyladénine.

3. Composition selon la revendication 1 pour l'utilisation selon la revendication 1, dans laquelle l'inhibiteur de la PI3K est choisi dans le groupe constitué par une molécule d'ARNi, un ribozyme, un acide nucléique antisens, un polynucléotide chimère ADN/ARN, et un vecteur l'exprimant.

4. Composition selon l'une quelconque des revendications précédentes pour l'utilisation selon l'une quelconque des revendications précédentes, dans laquelle le médicament qui supprime la GST-π est choisi dans le groupe constitué par une molécule d'ARNi, un ribozyme, un acide nucléique antisens, un polynucléotide chimère ADN/ARN, et un vecteur l'exprimant.


### SEQUENCE LISTING

<110> Nitto Denko Corporation
<120> Apoptosis-inducing agent
<130> PCT2512ND
<160> 2
<170> PatentIn version 3.1
<210> 1
   <211> 21
   <212> DNA
   <213> Artificial
<220>
   <223> GSTpi siRNA
<400> 1
   gggaggcaag accuucauut t 21
<210> 2
   <211> 27
   <212> DNA
   <213> Artificial
<220>
   <223> Scramble siRNA
<400> 2
   cgauucgcua gaccggcuuc auugcag 27